# EUROPÄISCHE PATENTSCHRIFT

(11) **EP 1 341 752 B1**
(45) Veröffentlichungstag und Bekanntmachung des Hinweises auf die Patenterteilung: **04.08.2004**
(21) Anmeldenummer: 01993598.0
(22) Anmeldetag: 30.10.2001
(51) Int. Cl.: C07C 255/40, C07D 319/18, C07F 7/08, G03C 1/815, A61K 7/42

(54) **NEUE INDANYLIDENVERBINDUNGEN**
NOVEL INDANYLIDENE COMPOUNDS
NOUVEAUX COMPOSES INDANYLIDENE

(30) Priorität: 10.11.2000 DE 10055940
(43) Veröffentlichungstag der Anmeldung: 10.09.2003
(60) Teilanmeldung: 04102032.2
(73) Patentinhaber: Symrise GmbH & Co. KG, 37603 Holzminden (DE)
(72) Erfinder: KOCH, Oskar, 37079 Göttingen (DE); DILK, Erich, 37603 Holzminden (DE); LANGNER, Roland, 37639 Bevern (DE); JOHNCOCK, William, 37671 Höxter (DE)
(74) Vertreter: Eisenführ, Speiser & Partner
(86) Internationale Anmeldenummer: PCT/EP2001/012516
(87) Internationale Veröffentlichungsnummer: WO 2002/038537

(56) Entgegenhaltungen:
- EP-A- 0 823 418
- WO-A-00/14172
- DATABASE CA [Online] CHEMICAL ABSTRACTS SERVICE, COLUMBUS, OHIO, US; CHIACCHIO, UGO ET AL: "Novel approach to the ring-opening of 4-isoxazolines: one-pot synthesis of.alpha.,.beta.-enones" retrieved from STN Database accession no. 116:128741 CA XP002190251 & TETRAHEDRON (1992), 48(1), 123-32 ,

## Beschreibung

Die Erfindung betrifft neue Indanylidenverbindungen, ein Verfahren zu Herstellung von Indanylidenverbindungen und ihre Verwendung als UVA-Filter.

Indanylidenverbindungen, die UV-absorbierende Eigenschaften haben, sind aus der EP-A 823 418 bereits bekannt. Die in der EP-A 823 418 vorbeschriebenen Indanylidenverbindungen haben jedoch eine für die Anwendung zu geringe Photostabilität.

In der WO 00/14172 werden u.a. Indanylidenverbindungen der Formel 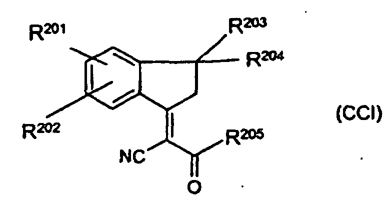 offenbart, wobei R²⁰¹ und R²⁰² unabhängig voneinander für C₁- bis C₈-Alkoxy, R²⁰³ und R²⁰⁴ unabhängig voneinander H oder C₁- bis C₄-Alkyl und R²⁰⁵ für C₁- bis C₁₈-Alkyl oder C₁- bis C₁₈-Alkoxy stehen. Die Verbindungen dienen als Gelbfilter für elektrochrome Medien.

Gemäß einem ersten Aspekt der Erfindung enthalten kosmetische Mittel zum Schutz von Haut und Haar Indanylidenverbindungen der Formel 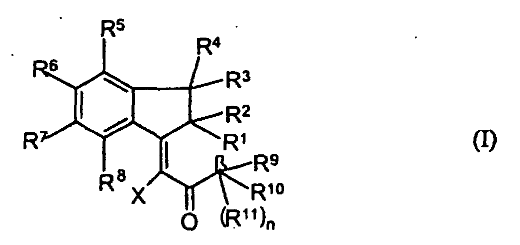 worin
- R¹ bis R⁴: unabhängig voneinander Wasserstoff, C₁-C₂₀-Alkyl oder C₅-C₁₀-Cycloalkyl bedeuten, mit der Maßgabe, dass auch zwei Substituenten an benachbarten C-Atomen zusammen eine gegebenenfalls substituierte C₁-C₄-Alkylengruppe bedeuten können;
weiterhin unabhängig voneinander C₂-C₂₀-Alkyl, in der mindestens eine Methylengruppe durch Sauerstoff, C₃-C₂₀-Alkenyl, C₃-C₂₀-Alkinyl oder einer Gruppe S ersetzt sein kann, bedeuten können,
wobei S eine Silan-, eine Oligosiloxan- oder eine Polysiloxan-Gruppe sein kann;
- R⁵ bis R⁸: unabhängig voneinander Wasserstoff C₁-C₂₀-Alkyl oder C₅-C₁₀-Cycloalkyl oder C₁ -C₂₀-Alkoxy, C₅-C₁₀-Cycloalkoxy, Hydroxy, Acetoxy, Acetamino, Carboxy, Carbalkoxy oder Carbamoyl bedeuten,
zusätzlich zwei Substituenten von R⁵ bis R⁸ an benachbarten C-Atomen zusammen einen 5-7-gliedrigen Ring bilden können, der bis zu drei Heteroatome, insbesondere Sauerstoff oder Stickstoff enthält, wobei die ' Ringatome durch exocyclisch doppelt gebundenen Sauerstoff (Ketogruppe) substituiert sein können,
weiterhin im Fall von Alkoxy unabhängig voneinander C₂-C₂₀-Alkyl, in der mindestens eine Methylengruppe durch Sauerstoff, C₃-C₂₀-Alkenyl, C₃-C₂₀ Alkinyl oder einer Gruppe S ersetzt sein kann, bedeuten können,
wobei S eine Silan-, eine Oligosiloxan- oder eine Polysiloxan-Gruppe sein kann;
- X: für Cyano, CON(R)₂ oder CO₂R, wobei R für Wasserstoff oder C₁-C₈-Alkyl steht;
- n: für 1 oder 0 steht;
- R⁹ bis R¹¹: im Fall von n=1 Wasserstoff, C₁-C₂₀-Alkyl oder C₅-C₁₀-Cycloalkyl, Aryl oder Heteroaryl bedeuten können,
zusätzlich zwei Substituenten von R⁹ bis R¹¹ gemeinsam mit dem β-Atom einen 3-7-gliedrigen Ring, der bis zu drei Heteroatome, insbesondere Sauerstoff oder Stickstoff enthalten kann, bilden können,
weiterhin im Fall von n=0 R⁹ und R¹⁰ gemeinsam mit dem β-Atom einen Aryl oder Heteroaryl-Rest bedeuten,

Die Indanylidenverbindungen stellen eine überraschende Auswahl unter den aus der EP-A 823 418 bekannten Indanylidenverbindungen dar. Sie haben eine deutlich höhere Photostabilität als die in EP-A 823 418 genannten Verbindungen und eine hohe Kompatibilität mit anderen UV-Filtern, wie z.B. p-Methoxyzimtsäure-isooctylester.

Bevorzugt enthalten kosmetische Mittel zum Schutz von Haut und Haar Indanylidenverbindungen der Formel

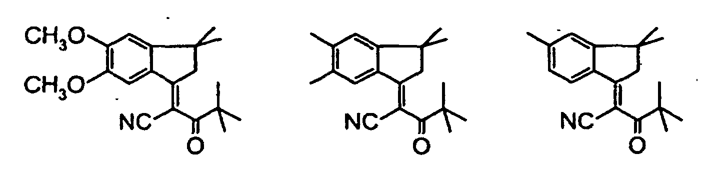

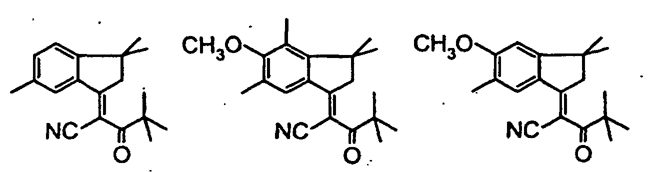

Insbesondere bevorzugt enthalten kosmetische Mittel zum Schutz von Haut und Haar Indanylidenverbindungen der Formel

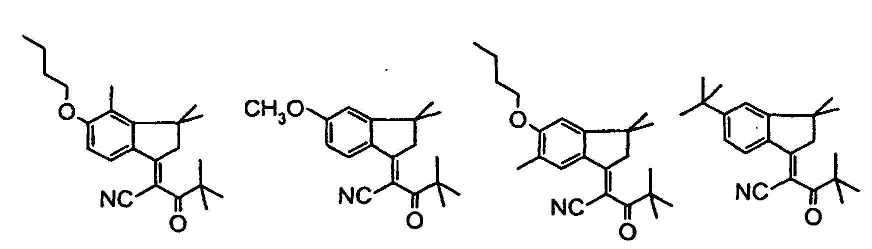

Im Einzelnen enthalten kosmetische Mittel zum Schutz von Haut und Haar die folgenden bevorzugten Indanylidenverbindungen: 2-(5,6-Dimethoxy-3,3-dimethyl-1-indanyliden)-4,4-dimethyl-3-oxo-pentansäurenitril, 2-(5-Methoxy-3,3,4,6-tetramethyl-1-indanyliden)-4,4-dimethyl-3-oxo-pentansäurenitril, 2-(3,3,5,6-tetramethyl-1-indanyliden)-4,4-dimethyl-3-oxo-pentansäurenitril, 2-(3,3,6-trimethyl-1-indanyliden)-4,4-dimethyl-3-oxo-pentansäurenitril, 2-(5,6-Ethylendioxo-3,3-dimethyl-1-indanyliden)-4,4-dimethyl-3-oxo-pentansäurenitril, 2-(5-Methoxy-3,3,6-trimethyl-1-indanyliden)-4,4-dimethyl-3-oxo-pentansäurenitril, 2-[(5-Methoxy-3,3-dimethyl-(2-methyl-3-(1,3,3,3-tetramethyl-1-(trimethyl-silyloxy)-disiloxanyl)-propyl)-indanyliden)]-4,4-dimethyl-3-oxo-pentansäurenitril und 2-(6-Acetoxy-3,3-dimethyl-5-methoxy-1-indanyliden)-4,4-dimethyl-3-oxo-pentansäurenitril.

Gemäß einem zweiten Aspekt der Erfindung werden die Indanylidenverbindungen der Formel (I) durch (Knoevenagel)-Kondensation von Verbindungen der Formel 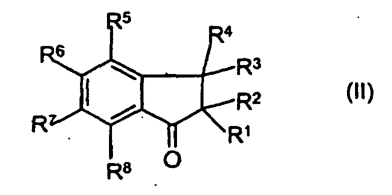 wobei
R¹ bis R⁸ die oben genannte Bedeutung haben,
mit Verbindungen der Formel 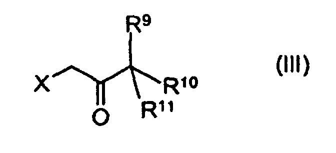 wobei
R⁹ bis R¹¹ und X die oben genannte Bedeutung haben,
hergestellt.

Die hierfür verwendeten Indanone können durch Friedel-Crafts-Reaktionen von (substituierten) Acrylsäureestern mit (substituierten) Aromaten oder im Fall von Hydroxysubstituenten durch Fries'sche Umlagerung entsprechender Phenolester hergestellt werden (F.-H. Marquardt, Helv. Chim. Acta 159, 1476 (1965)).

Die Herstellung der erfindungsgemäßen Indanylidenverbindungen kann beispielsweise wie folgt durchgeführt werden:

Man kondensiert die o.a. Indanone mit eqimolaren Mengen Pivaloylacetonitril unter der Katalyse von Ammoniumacetat gemäß den Bedingungen einer Knoevenagel-Kondensation.

Die Herstellung kann durch das folgende Reaktionsschema erläutert werden:

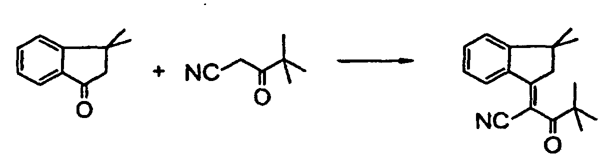

Die erfindungsgemäßen Indanylidenverbindungen können als UV-Absorber, z.B. in kosmetischen Mitteln, insbesondere zum Schutz vor akuten (Sonnenbrand) sowie chronischen (frühzeitiger Hautalterung) Hautschäden besonders in Sonnenschutzmitteln, Tagespflegeprodukten und Haarpflegeprodukten verwendet werden.

Ein dritter Aspekt der Erfindung betrifft neue Indanylidenverbindungen der folgenden Formeln

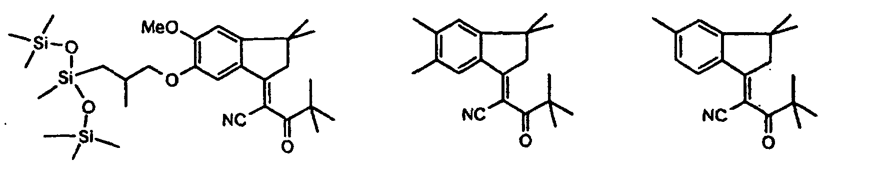

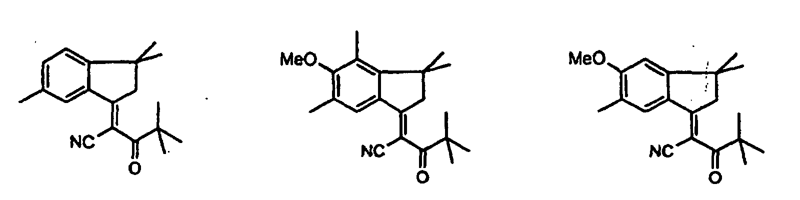

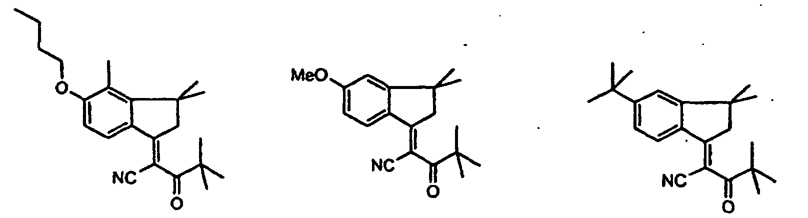

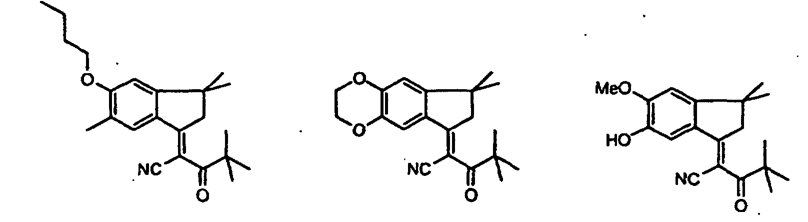

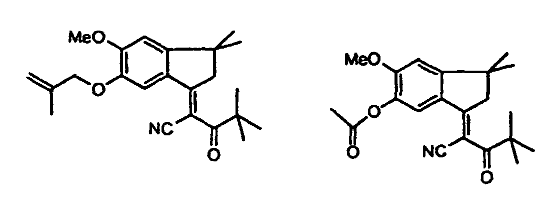

Die erfindungsgemäßen Indanylidenverbindungen können einzeln oder in Mischung in den entsprechenden Zubereitungen eingesetzt werden; man kann sie auch in Kombination mit UV-Absorbem anderer Substanzklassen oder auch mit diesen in beliebigen Gemischen untereinander einsetzen. Beispielsweise seien die folgenden UV-Absorber genannt:
- p-Aminobenzoesäure
- p-Aminobenzoesäureethylester (25 Mol) ethoxyliert
- p-Dimethylaminobenzoesäure-2-ethylhexyiester
- p-Aminobenzoesäureethylester (2 Mol) N-propoxyliert
- p-Aminobenzoesäureglycerinester
- Salicylsäure-homomenthylester (Homosalate) (Neo Heliopan®HMS)
- Salicylsäure-2-ethylhexylester (Neo Heliopan®OS)
- Triethanolaminsalicylat
- 4-Isopropylbenzylsalicylat
- Anthranilsäurementhylester (Neo Heliopan®MA).
- Diisopropylzimtsäureethylester
- p-Methoxyzimtsäure-2-ethylhexylester (Neo Heliopan®AV)
- Diisopropylzimtsäuremethylester
- p-Methoxyzimtsäureisoamylester (Neo Heliopan®E 1000)
- p-Methoxyzimtsäure-diethanolaminsalz
- p-Methoxyzimtsäure-isopropylester
- 2-Ethylhexyl-2-cyano-3,3-diphenylacrylat (Neo Heliopan®303)
- Ethyl-2-cyano-3,3'-diphenylacrylat
- 2-Phenylbenzimidazolsulfonsäure und Salze (Neo Heliopan®Hydro)
- 3-(4'-Trimethylammonium)-benzyliden-bornan-2-on-methylsulfat
- Terephthalyliden-dibornansulfonsäure und Salze (Mexoryl®SX)
- 4-t-Butyl-4'-methoxy-dibenzoylmethan (Avobenzon) / (Neo Heliopan®357)
- β-Imidazol-4(5)-acrylsäure (Urocaninsäure)
- 2-Hydroxy-4-methoxybenzophenon (Neo Heliopan®BB)
- 2-Hydroxy-4-methoxybenzophenon-5-sulfonsäure
- Dihydroxy-4-methoxybenzophenon
- 2,4-Dihydroxybenzophenon
- Tetrahydroxybenzophenon
- 2,2'-Dihydroxy-4,4'-dimethoxybenzophenon
- 2-Hydroxy-4-n-octoxybenzophenon
- 2-Hydroxy-4-methoxy-4'-methylbenzophenon
- 3-(4'-Sulfo)benzyliden-bornan-2-on und Salze
- 3-(4'-Methylbenzyliden)-d,1-campher (Neo Heliopan®MBC)
- 3-Benzyliden-d,1-campher
- 4-Isopropyldibenzoylmethan
- 2,4,6-Trianilino-(p-carbo-2'-ethylhexyl-1'-oxy)-1,3,5-triazin
- Phenylen-bis-benzimidazyl-tetrasulfonsäure-dinatriumsalz (Neo Heliopan®AP)
- 2,2'-(1,4-Phenylen)-bis-(1H-benzimidazol-4,6-disulfonsäure), Mononatriumsalz
- N-[(2 und 4)-[2-(oxoborn-3-yliden)methyl]benzyl]-acrylamid-Polymer
- Phenol, 2-(2H-benzotriazol-2-yl)-4-methyl-6-(2-methyl-3(1,3,3,3-tetramethyl-1-(trimethylsilyl)-oxy)-disiloxyanyl)-propyl), (Mexoryl®XL)
- 4,4'-[(6-[4-(1,1-Dimethyl)-aminocarbonyl)-phenylamino]-1,3,5-triazin-2,4-diyl)diimino]-bis-(benzoesäure-2-ethylhexylester) (Uvasorb®HEB)
- 2,2'-Methylen-bis-(6-(2H-benztriazol-2-yl)-4-1,1,3,3-tetramethylbutyl)-phenol), (Tinosorb®M)
- 2,4-bis-[4-(2-ethylhexyloxy)-2-hydroxyphenyl]-1,3,5-triazin
- Benzylidenmalonat-Polysiloxan (Parsol®SLX)
- Glyceryl-ethylhexanoat-dimethoxycinnamat
- Dinatrium-2,2'-dihydroxy-4,4'-dimethoxy-5,5'-disulfo-benzophenon
- Dipropylenglykolsalicylat
- Natrium-hydroxymethoxybenzophenon-sulfonat
- 4,4',4-(1,3,5-Triazin-2,4,6-triyltriimino)-tris-benzoesäure-tris(2-ethylhexylester) (Uvinul®T150)
- 2,4-Bis-[{(4-(2-Ethyl-hexyloxy)-2-hydroxy}-phenyl]-6-(4-methoxyphenyl)-1,3,5-triazin, (Tinosorb®S)
- 2,4-Bis-[{(4-(3-sulfonato)-2-hydroxy-propyloxy)-2-hydroxy}-phenyl]-6-(4-methoxyphenyl)-1,3,5-triazin-Natriumsalz
- 2,4-Bis-[{(3-(2-Propyloxy)-2-hydroxy-propyloxy)-2-hydroxy}-phenyl]-6-(4-methoxy-phenyl)-1,3,5-triazin
- 2,4-Bis-[{4-(2-Ethyl-hexyloxy)-2-hydroxy}-phenyl]-6-[4-(2-methoxyethylcarbonyl)-phenylamino]-1,3,5-triazin
- 2,4-Bis-[{4-(3-(2-propyloxy)-2-hydroxy-propyloxy)-2-hydroxy}-phenyl]-6-[4-(2-ethylcarboxyl)-phenylamino]-1,3,5-triazin
- 2,4-Bis-[{4-(2-Ethyl-hexyloxy)-2-hydroxy}-phenyl]-6-(1-methyl-pyrrol-2-yl-)-1,3,5-triazin
- 2,4-Bis-[{4-tris-(trimethylsiloxy-silylpropyloxy)-2-hydroxy}-phenyl]-6-(4-methoxyphenyl)-1,3,5-triazin
- 2,4-Bis-[{4-(2''-Methylpropenyloxy)-2-hydroxy}-phenyl]-6-(4-methoxyphenyl)-1,3,5-triazin
- 2,4-Bis-[{4-(1',1',1',3'5',5',5'-Heptamethylsiloxy-2"-methyl-propyloxy)-2-hydroxy}-phenyl]-6-(4-methoxyphenyl)-1,3,5-triazin

Besonders geeignete UV-Absorber sind:
- p-Aminobenzoesäure
- 3-(4'-Trimethylammonium)-benzyliden-bornan-2-on-methylsulfat
- Salicylsäure-homomenthylester (Neo Heliopan®HMS)
- 2-Hydroxy-4-methoxy-benzophenon (Neo Heliopan®BB)
- 2-Phenylbenzimidazolsulfonsäure (Neo Heliopan®Hydro)
- Terephthalyliden-dibornansulfonsäure und Salze (Mexoryl®SX)
- 4-tert.-Butyl-4'-methoxydibenzoylmethan (Neo Heliopan®357)
- 3-(4'-Sulfo)benzyliden-bornan-2-on und Salze
- 2-Ethylhexyl-2-cyano-3,3-diphenylacrylat (Neo Heliopan®303)
- N-[(2 und 4)-[2-(oxoborn-3-yliden)methyl]benzyl]-acrylamid-Polymer
- p-Methoxyzimtsäure-2-ethylhexylester (Neo Heliopan®AV)
- p-Aminobenzoesäure-ethylester (25 Mol) ethoxyliert
- p-Methoxyzimtsäure-isoamylester (Neo Heliopan®E1000)
- 2,4,6-Trianilino-(p-carbo-2'-ethylhexyl-1'-oxy)-1,3,5-triazin (Uvinul®T150)
- Phenol, 2-(2H-benzotriazol-2-yl)-4-methyl-6-(2-methyl-3(1,3,3,3-tetramethyl-1-(trimethylsilyl)-oxy)-disiloxyanyl)-propyl), (Mexoryl®XL)
- 4,4'-[(6-[4-(1,1-Dimethyl)-aminocarbonyl)-phenylamino]-1,3,5-triazin-2,4-diyl)-diimino]-bis-(benzoesäure-2-ethylhexylester), (UvasorbHEB)
- 3-(4'-Methylbenzyliden)-d,l-campher (Neo Helipan®MBC)
- 3-Benzylidencampher
- Salicylsäure-2-ethylhexylester (Neo Helipan®OS)
- 4-Dimethylaminobenzoesäure-2-ethylhexylester (Padimate O)
- Hydroxy-4-methoxy-benzophenon-5-sulfonsäure und Na-Salz
- 2,2'-Methylen-bis-(6-(2H-benztriazol-2-yl)-4-1,1,3,3-tetramethylbutyl)-phenol), (Tinosorb®M)
- Phenylen-bis-benzimidazyl-tetrasulfonsäure-dinatriumsalz (Neo Heliopan®AP)
- 2,4-Bis-[{(4-(2-Ethyl-hexyloxy)-2-hydroxy}-phenyl]-6-(4-methoxyphenyl)-1,3,5-triazin, (Tinosorb®S)
- Benzylidenmalonat-Polysiloxan (Parsol®SLX)
- Menthylanthranilat (Neo Heliopan®MA)

Es kann auch von Vorteil sein, polymer-gebundene oder polymere UV-Absorber in Zubereitungen gemäß der vorliegenden Erfindung zu verwenden, insbesondere solche, wie sie in WO-A-92/20690 beschrieben werden. Ebenso ist die Kombination der erfindungsgemäßen Indanylidenverbindungen mit feinteiligen anorganischen und organischen Pigmenten, wie z.B. Titandioxid, Zinkoxid und Eisenoxid bzw. Tinosorb®M, in Sonnenschutz- und Tagespflegeprodukten mit UV-Schutz möglich.

Die Aufzählung der genannten UV-Filter, die im Sinne der vorliegenden Erfindung eingesetzt werden können, soll selbstverständlich nicht limitierend sein.

Die Gesamtmenge aller (einfach und mehrfach sulfonierter) wasserlöslichen UV-Filtersubstanzen in den fertigen kosmetischen oder dermatologischen Zubereitungen, beispielsweise an Phenylen-bis-benzimidazyl-tetrasulfonsäure-dinatriumsalz bzw. deren Salzen und/oder die entsprechende Disulfonsäure bzw. deren Salzen und/oder 2-Phenylbenzimidazol-5-sulfonsäure bzw. deren Salzen und/oder 2-Hydroxy-4-methoxybenzophenon-5-sulfonsäure bzw. deren Salzen und/oder 4-(2-Oxo-3-bornylidenmethyl)-benzolsulfonsäure bzw. deren Salzen und/oder 2-Methyl-5-(2-oxo-3-bornyliden-methyl)-benzolsulfonsäure bzw. deren Salzen und/oder Benzol-1,4-di-(2-oxo-3-bornylidenmethyl)-10-sutfonsäure bzw. deren Salzen, wird vorteilhaft aus dem Bereich von 0,1 bis 10,0 Gew.-%, bevorzugt 0,5 bis 6,0 Gew.-% gewählt, bezogen auf das Gesamtgewicht der Zubereitungen, falls die Gegenwart dieser Substanzen erwünscht ist.

Die Gesamtmenge an öllöslichen UV-Filtersubstanzen in den fertigen kosmetischen oder dermatologischen Zubereitungen, beispielsweise an 4,4',4''-(1,3,5-Triazin-2,4,6-triyltriimino)-tris-benzoesäure-tris-(2-ethylhexylester) und/oder 4-tert.-Butyl-4'-methoxy-dibenzoylmethan und/oder 4-Methylbenzylidencampher und/oder Octyldimethyl-p-Aminobenzoesäure und/oder Mexoryl®XL und/oder Uvasorb®HEB und/oder Tinosorb®S und/oder Benzophenon-3 und/oder Parsol®SLX und/oder Neo Heliopan®MA wird vorteilhaft aus dem Bereich von 0,1 bis 10,0 Gew.-%, bevorzugt 0,5 bis 6,0 Gew.-% gewählt, bezogen auf das Gesamtgewicht der Zubereitungen, falls die Gegenwart dieser Substanzen erwünscht ist.

Die Gesamtmenge an 2-Ethylhexyl-p-methoxy-cinnamaten und/oder p-Methoxy-zimtsäureisoamylester in den fertigen kosmetischen oder dermatologischen Zubereitungen wird vorteilhaft aus dem Bereich von 0,1 bis 15,0 Gew.-%, bevorzugt 0,5 bis 7,5 Gew.-% gewählt, bezogen auf das Gesamtgewicht der Zubereitungen, falls die Gegenwart dieser Substanzen erwünscht ist.

Die Gesamtmenge an Ethylhexyl-2-cyano-3,3-diphenylacrylat in den fertigen kosmetischen oder dermatologischen Zubereitungen wird, falls die Gegenwart dieser Substanz erwünscht ist, vorteilhaft aus dem Bereich von 0,1 bis 15,0 Gew.-%, bevorzugt 0,5 bis 10,0 Gew.-% gewählt, bezogen auf das Gesamtgewicht der Zubereitungen.

Die Gesamtmenge an einem oder mehreren Salicylsäurederivaten in den fertigen kosmetischen oder dermatologischen Zubereitungen wird vorteilhaft aus dem Bereich von 0,1 bis 15,0 Gew.-%, bevorzugt 0,5 bis 10,0 Gew.-% gewählt, bezogen auf das Gesamtgewicht der Zubereitungen. Wenn Ethylhexylsalicylat gewählt wird, ist es von Vorteil, dessen Gesamtmenge aus dem Bereich von 0,1 bis 5,0 Gew.-% zu wählen. Wenn Homomenthylsalicylat gewählt wird, ist es von Vorteil, dessen Gesamt-menge aus dem Bereich von 0,1 bis 10,0 Gew.-% zu wählen.

Die erfindungsgemäßen Indanylidenverbindungen eignen sich auch in besonderem Maße zur Photostabilisierung von UV-Absorbern mit geringer UV-Lichtstabilität. Insbesondere gelingt die Photostabilisierung der sehr lichtinstabilen Verbindungen der Dibenzoylmethane, z.B. tert.-Butyl-4'-methoxydibenzoylmethan.

Eine weitere lichtstabile UV-Filterkombination wird erreicht durch Einsatz von 0,1 bis 10 Gew.-%, bevorzugt 1 bis 10 Gew.-% p-Methoxyzimtsäura-ethylhexylester oder -isoamylester mit 0,1 bis 10 Gew.-%, bevorzugt 1 bis 6 Gew.-% der Verbindung der Formel I, bevorzugt im Verhältnis 1:1.

Die Kombinationen von p-Methoxyzimtsäureestern und Dibenzoylmethanderivaten und Verbindungen der Formel I lassen sich lichtstabil formulieren durch Einsatz von z.B. 0,1 bis 5 Gew.-%, bevorzugt 1 bis 3 Gew.-% 4-tert.-Butyl-4'-methoxydibenzoylmethan, 0,1 bis 10 Gew.-%, bevorzugt 1 bis 7,5 Gew.-% p-Methoxyzimtsäureethylhexyl- oder isoamylester und mindestens 0,2 Gew.-%, bevorzugt 1 bis 6 Gew.-% der Verbindungen der Formel I, bevorzugt im Verhältnis 1 Teil Dibenzoylmethanderivat, 2 Teile p-Methoxyzimtsäureester und 2 Teile der erfindungsgemäßen Indanylidenverbindungen.

Vorteilhaft ist weiterhin zu dieser Dreierkombination einen oder weitere sehr photostabile UV-Absorber, wie z.B. Methylbenzylidencampher, 2-Ethylhexyl-2-cyano-3,3'-diphenylacrylat, Octyltriazon, Uvasorb®HEB, Tinosorb®S, Tinosorb®M, Ethylheoylsalicylat, Homomenthylsalicylat sowie Phenylenbenzimidazolsulfonsäure oder Phenylen-bis-benzimidazol-tetrasulfonsäure-dinatriumsalz, Mexoryl®SX, Mexoryl®XL oder Parsol®SLX hinzuzusetzen.

Weiterhin wird in kosmetischen Zubereitungen überraschenderweise durch die Verwendung von Indanyliden-Derivaten der Formel I in Kombination mit anderen UV-Filtern eine synergistische Erhöhung des Sonnenschutzfaktors erreicht. Beispiele für eine synergistische Erhöhung des Sonnenschutzfaktors sind kosmetische Emulsionen, die sowohl eine Verbindung der Formel I als auch Ethylhexylmethoxycinnamat oder Octocrylene, oder eine Kombination von Verbindung der Formel I mit Ethylhexylmethoxycinnamat und 2-Phenylbenzimidazolsulfonsäure, oder Ethylhexyl-methoxycinnamat und Methylbenzylidencampher, oder Ethylhexyl-methoxycinnamat und 4-t-Butyl-4'-methoxy-dibenzoylmethan, oder Neo Heliopan® AP und Ethyl-hexylmethoxycinnamat, oder eine Kombination von Verbindung der Formel I mit Octocrylene, Methylbenzylidencampher und Zinkoxid enthalten. Ebenfalls weisen Kombinationen von Verbindung der Formel I mit Dibenaoylmethanen, Methyl-benzylidencampher, 2-Phenylbenzimidazolsulfonsäure, Neo Heliopan®AP, Mexoryl® SX, Mexoryl®XL, Parsol®SLX, Tinosorb®S, Tinosorb®M, Uvinul®T150, Uvasorb® HEB sowie mikrofeinen Pigmenten, Zinkoxid und Titandioxid, synergistische Erhöhungen des Sonnenschutzfaktors auf. Die genannten UV-Filter-Kombinationen sind beispielhaft angeführt und nicht auf die oben genannten Kombinationen beschränkt zu verstehen. So können alle auf Seiten 8/9 bereits genannten besonders geeigneten UV-Absorber sowie alle in den nachfolgenden Veröffentlichungen zugelassenen UV-Filter mit Verbindungen der Formel I oder o.a. Kombinationen, einzeln oder in beliebigen Mischungen, kombiniert eingesetzt werden.
- USA:: Food and Drug Administration (FDA). Veröffentlichung in Monographie für Sunscreen Drug Products for Over-The-Counter Human Use.
- Europa:: Richtlinie 76/768 EWG des Rates zur Angleichung der Rechtsvorschriften der Mitgliedsstaaten über kosmetische Mittel an den technischen Fortschritt. Veröffentlichungen im Official Journal of European Communities.
- Japan:: Veröffentlichung der Kosmetik-Richtlinie des Ministry of Health and Welfare (MHW).
- Deutschland:: Veröffentlichung in der Verordnung über kosmetische Mittel gemäß dem Lebensmittel- und Bedarfsgegenstände-Gesetz (LMBG).
- Australien:: Registrierung durch Therapeutic Goods Administration (TGA) und Veröffentlichung im Australian Register of Therapeutic Goods (ARTG).

Üblicherweise werden durch diese Kombinationen eine synergistische Erhöhung des UV-Sonnenschutzfaktors erzielt.

Die Kombination von Verbindungen der Formel I mit UV-A-Absorbern, besonders UV-A-II-Absorbern, ergibt einen umfassenden Schutz gegen die UV-A-Strahlung (320-400 nm). Insbesondere eine Kombination von Verbindungen der Formel I mit Neo Heliopan® AP (UV-AII-Absorber) ist für eine breite UV-A-Schutzleistung zu nennen. Weitere UV-A-Filter, die in Kombination mit Verbindungen der Formel I allein oder in Kombination von Verbindungen der Formel I und Neo Heliopan®AP bevorzugt werden, sind Mexoryl®SX, Mexoryl®XL, Tinosorb®M Tinosorb®S, Benzophenon-3, Benzophenon-4, Neo Heliopan®357, Neo Heliopan®MA.

Für eine optimale Breitbandschutzleistung gegen UV-A- und UV-B-Strahlung sind vorgenannte Kombinationen mit allen UV-B-Filtern und Mischungen aus diesen Filtern zu kombinieren (vgl. Liste auf Seiten 6-9). Bevorzugt geeignet sind Neo Heliopan®AV, Neo Heliopan®E1000, Neo Heliopan®Hydro, Neo Heliopan®MBC, Neo Heliopan®303, Neo Heliopan®OS, Neo Heliopan®HMS, Uvinul®T150, Uvasorb®HEB, Dimethylaminobenzoesäure-ethylhexylester.

Durch Kombination von Verbindungen der Formel I mit Neo Heliopan®AP und einem UV-B-Filter, z.B. Ethylhexylmethoxycinnamat oder UV-B-Filtergemischen sowie beschichteten oder unbeschichteten feindispersen Metalloxiden wie z.B. Zinkoxid, Titandioxid wird eine UV-Breitband-Schutzleistung mit, einer kritischen Wellenlänge λ_{crit.}> 380 nm erzielt *(vgl. hierzu Diffey in Int. J. Cosm. Science 16, 47 (1994))*.

Weiterhin lassen sich die erfindungsgemäßen Indanylidenverbindungen allein oder mit anderen UV-Absorbern, die für den Schutz techriischer Produkte eingesetzt werden, kombinieren.

Beispiele solcher UV-Absorber sind Verbindungen aus der Reihe der Benzotriazole, Benzophenone, Triazine, Zimtsäureester sowie Oxalanilide.

Die erfindungsgemäßen Indanylidenverbindungen sind kristallin und müssen in kosmetischen Zubereitungen ausreichend gelöst werden, um das Problem der Rekristallisation nach längerer Lagerungszeit zu vermeiden. Eine ausreichende Menge der üblicherweise in kosmetischen Zubereitungen eingesetzten Ölkomponenten, flüssiger öllöslicher UV-Absorber oder Alkohole, z. B. Ethanol, Isopropanol oder 1-Butanol, ist zur Vermeidung der Rekristallisation erforderlich. Besonders bevorzugt ist der Einsatz folgender Ölkomponenten und/oder UV-Absorber zur Erzielung einer ausreichenden Löslichkeit von Verbindungen der erfindungsgemäßen Indanylidenverbindungen:

Ethylhexylmethoxycinnamat, Isoamyl-methoxycinnamat, Octocrylen, Ethylhexylsalicylat, Homosalat, Menthylanthranilat, Padimate O, Diisopropyladipat, C₁₂₋₁₅-Alkyl-benzoat (Witconol TN), Butylenglykol-dicaprylat/-dicaprat (Miglyol 8810), Cocoglyceride (Myritol 331), Capryl/capr.-triglyceride (Miglyol 812), Cetearyl-isononanat (Cetiol SN), PVP/Hexadecen-Copolymer (Unimer U151), Adipinsäure/Diethylenglykol/Isononausäure-Copolymer (Lexorez 100), Propylenglykoldicaprylat/ Dicaprat (Myritol PC), Hexyllaurat (Cetiol A), Dicaprylether (Cetiol OE), Diethylhexyl-naphthalat (Hallbrite®TQ), Butyloctylsalicylat (Hallbrite®BHB), Dibutyladipat (Cetiol B), Triethylcitrat (Hydagen CAT), Propylenglykol-dibenzoat (Finsolv PG 22), Tributylcitrat, Dioctylmalat (Ceraphyl 45), Dipropylenglykoldibenzoat (Benzoflex 245), Acetyl-tributylcitrat (Citroflex A-4), Acetyl-triethylcitrat (Citroflex A-2). Die Aufzählung der genannten Öle, die im Sinne der vorliegenden Erfindung eingesetzt werden können, soll selbstverständlich nicht limitierend sein.

Die Einsatzmenge aller Ölkomponenten in kosmetischen Emulsionen mit Verbindungen der Formel I ist 0,5 bis 30 Gew.-%, bevorzugt 2 bis 15 Gew.-%. Alle genannten Ölkomponenten bzw. flüssigen öllöslichen UV-Filter sind ausgezeichnete Lösungsmittel für alle kristallinen öllöslichen UV-Absorber.

Sehr nachteilig ist, wenn UV-Absorber auf Kleidungsstücken nicht mehr auswaschbare Flecken hinterlassen. Insbesondere ist von dem UV-A-Absorber tert.-Butylmethoxydibenzoylmethan bekannt, dass er nicht mehr auswaschbare Flecken auf Textilien erzeugt. Diesen Nachteil haben die erfindungsgemäßen Indanylidenverbindungen nicht, da eine Fleckenbildung auf Textilien sehr gut auswaschbar ist.

Sonnenschutzprodukte sollen wasserfest sein, damit ein ausreichender UV-Schutz für den Anwender, insbesondere Kinder, beim Schwimmen oder Baden gewährleistet ist. Verbindungen der erfindungsgemäßen Indanylidenverbindungen erfüllen diese Anforderungen in besonderem Maße. In einer O/W-Emulsion mit 3 Gew.-% einer Verbindung der erfindungsgemäßen Indanylidenverbindungen wurden 97 % Substautivität des UV-Absorbers nach dem Waschen gemessen und in einer W/O-Emulsion 95%. Weiterhin kann die Wasserfestigkeit von Sonnenschutzprodukten mit wasserlöslichen, einfach oder mehrfach sulfonierten UV-Filtern wie z.B. Neo Heliopan®AP, Mexoryl®SX, Benzophenon-4, Neo Heliopan®Hydro und den auf Seiten 6-9 aufgeführten öllöslichen UV-Absorbern durch Kombination mit Verbindungen der Formel I signifikant erhöht werden.

Es kann ferner von erheblichen Vorteil sein, die erfindungsgemäß genannten UV-Absorber mit chelatisierenden Substanzen, wie sie z.B. in EP-A 496 434, EP-A 313 305 und WO-94/04128 aufgeführt sind, oder mit Polyasparaginsäure und Ethylendiamintetramethyl-phosphonsäuresalzen zu kombinieren.

Kosmetische und dermatologische Formulierungen im Sinne der Erfindung enthalten einen oder mehrere übliche UV-A-, UV-B- und/oder Breitbandfilter als Einzelsubstanzen oder in beliebigen Gemischen untereinander, in der Lipidphase und/oder in der wässrigen Phase. Sie sind in jeglicher Hinsicht befriedigende Produkte, welche sich erstaunlicherweise durch eine hohe UV-A-Schutzleistung bzw. hohen Sonnenschutzfaktor auszeichnen.

Gegenstand der Erfindung ist ferner die Verwendung der erfindungsgemäßen UV-Absorber in Kombination mit herkömmlichen UV-Absorbern zur Verstärkung des Schutzes vor schädigender UV-Strahlung über das Maß des Schutzes, der bei Einsatz gleicher Mengen herkömmlicher oder der erfindungsgemäßen UV-Filter allein erzielt wird (synergistischer Effekt).

Die Gesamtmenge an UV-Filtersubstanzen (UV-A-, UV-B- und/ oder Breitbandfilter) in den fertigen kosmetischen oder dermatologischen Zubereitungen, sei es als Einzelsubstanz oder in beliebigen Gemischen untereinander, wird vorteilhaft aus dem Bereich 0,1 bis 30 Gew.-%, bevorzugt 0,1 bis 10,0 Gew.-%, insbesondere 0,5 bis 5,0 Gew.-% gewählt, bezogen auf das Gesamtgewicht der Zubereitungen.

Erfindungsgemäße kosmetische und dermatologische Zubereitungen enthalten ferner vorteilhaft, wenngleich nicht zwingend, anorganische Pigmente auf Basis von feindispersen Metalloxiden und/oder anderen in Wasser schwerlöslichen oder unlöslichen Metallverbindungen, insbesondere der Oxide des Titans (TiO₂), Zinks (ZnO), Eisens (z.B. Fe₂O₃), Zirkoniums (ZrO₂), Siliciums (SiO₂), Mangans (z.B. MhO), Aluminiums (Al₂O₃), Cers (z.B. Ce₂O₃), Mischoxiden der entsprechenden Metalle sowie Abmischungen aus solchen Oxiden. Diese Pigmente sind röntgenamorph oder nichtröntgenamorph. Besonders bevorzugt handelt es sich um Pigmente auf der Basis von TiO₂.

Röntgenamorphe Oxidpigmente sind Metalloxide oder Halbmetalloxide, welche bei Röntgenbeugungsexperimenten keine oder keine erkenntliche Kristallstruktur erkennen lassen. Oftmals sind solche Pigmente durch Flammenreaktion erhältlich, beispielsweise dadurch, dass ein Metall- oder Halbmetallhalogenid mit Wasserstoff und Luft (oder reinem Sauerstoff) in einer Flamme umgesetzt wird.

In kosmetischen, dermatologischen oder pharmazeutischen Formulierungen werden röntgenamorphe Oxidpigmente als Verdickungs- und Thixotropierungsmittel, Fließhilfsmittel zur Emulsions- und Dispersionsstabilisierung und als Trägersubstanz (beispielsweise zur Volumenerhöhung von feinteiligen Pulvern oder Pudern) eingesetzt. Bekannte und in der kosmetischen oder dermatologischen Galenik oftmals verwendete röntgenamorphe Oxidpigmente sind beispielsweise hochreines Siliciumoxid. Bevorzugt werden hochreine, röntgenamorphe Siliciumdioxid-pigmente mit einer Partikelgröße im Bereich von 5 bis 40 nm und einer aktiven Oberfläche (BET) im Bereich von 50 bis 400 m²/g, bevorzugt 150 bis 300 m²/g, wobei die Partikel als kugelförmige Teilchen mit sehr einheitlicher Abmessung anzusehen sind. Makroskopisch sind die Siliciumdioxidpigmente als lockere, weiße Pulver erkenntlich. Siliciumdioxidpigmente werden im Handel unter dem Namen Aerosil® (CAS-Nr. 7631-85-9) oder Carb-O-Sil vertrieben Vorteilhafte Aerasil®-Typen sind beispielsweise Aerosil®0X50, Aerosil®130, Aerosil®150, Aerosil®200, Aerosil®300, Aerosil®380, Aerosil®MQX 80, Aerosil® MOX 170, Aerosil®COK 84, Aerosil® R 202, Aerosil®R 805, Aerosil®R 812, Aerosil®R 972, Aerosil®R 974, Aerosil®R976.

Erfindungsgemäß enthalten kosmetische oder dermatologische Lichtschutzzubereitungen 0,1 bis 20 Gew.-%, vorteilhaft 0,5 bis 10 Gew.-%, ganz besonders bevorzugt 1 bis 5 Gew.-% röntgenamorphe Oxidpigmente.

Die nicht-röntgenamorphen anorganischen Pigmente liegen erfindungsgemäß vorteilhaft in hydrophober Form vor, d.h., dass sie oberflächlich wasserabweisend behandelt sind. Diese Oberflächenbehandlung kann darin bestehen, dass die Pigmente nach an sich bekannten Verfahren mit einer dünnen hydrophoben Schicht versehen werden. Eines solcher Verfahren besteht beispielsweise darin, dass die hydrophobe Oberflächenschicht nach einer Reaktion gemäß

n TiO₂ + m (RO)₃Si-R' → n TiO₂ (oberfl.)

erzeugt wird. n und m sind dabei nach Belieben einzusetzende stöchiometrische Parameter, R und R' die gewünschten organischen Reste. Beispielsweise in Analogie zu DE-A 33 14 742 dargestellte hydrophobisierte Pigmente sind von Vorteil.

Beispielsweise seien TiO₂-Pigmente genannt, wie sie unter der Handelsbezeichnung T805 von der Firma Degussa vertrieben werden. Bevorzugt sind auch TiO₂/Fe₂O₃-Mischoxide, wie sie beispielsweise unter der Handelsbezeichnung T817 ebenfalls von der Firma Degussa angeboten werden.

Die Gesamtmenge an anorganischen Pigmenten, insbesondere hydrophoben anorganischen Mikropigmenten in den fertigen kosmetischen oder dermatologischen Zubereitungen wird vorteilhaft aus dem Bereich von 0,1 bis 30 Gew.-%, bevorzugt 0,1 bis 10,0, insbesondere 0.5 bis 6.0 Gew.-% gewählt, bezogen auf das Gesamtgewicht der Zubereitungen.

Die erfindungsgemäßen kosmetischen und/oder dermatologischen Formulierungen können wie üblich zusammengesetzt sein und dem kosmetischen und/oder dermatologischen Sonnenschutz, ferner zur Behandlung, der Pflege und der Reinigung der Haut und/oder der Haare und als Schminkprodukt in der dekorativen Kosmetik dienen. Entsprechend können die erfindungsgemäßen Zubereitungen, je nach ihrem Aufbau, beispielsweise verwendet werden als Hautschutzcreme, Reinigungsmilch, Sonnenschutzlotion, Nährcreme, Tages- oder Nachtcreme usw. Es ist gegebenenfalls möglich und vorteilhaft, die erfindungsgemäßen Zubereitungen als Grundlage für pharmazeutische Formulierungen zu verwenden. Bevorzugt sind insbesondere solche kosmetischen und dermatologischen Zubereitungen, die in der Form eines Hautpflege- bzw. Schminkproduktes vorliegen. Typische Ausgestaltungen sind Cremes, Gele, Lotionen, alkoholische und wäßrig/alkoholische Lösungen, Emulsionen oder Stiftpräparate. Diese Mittel können ferner als weitere Hilfs- und Zusatzstoffe milde Tenside, Co-Emulgatoren, Überfettungsmittel, Perlglanzwachse, Konsistenzgeber, Verdickungsmittel, Polymere, Siliconverbindungen, Fette, Wachse, Stabilisatoren, biogene Wirkstoffe, Deowirkstoffe, Antischuppenmittel, Filmbildner, Quellmittel, Hydrotrope, Konservierungsmittel, Insektemepellentien, Bräunungsmittel, künstliche Selbstbräunungsmittel (z.B. Dihydroxyaceton), Solubilisatoren, Parfümöle, Farbstoffe, keimhemmende Mittel und dergleichen enthalten.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines kosmetischen Mittels zum Schutz von Haut und Haaren vorliegen. Vorteilhaft können diese zusätzlich zu erfindungsgemäß verwendeten UV-A, UV-B- und/oder Breitbandfiltern mindestens ein anorganisches Pigment, bevorzugt ein anorganisches Mikropigment, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate. Als nicht ionische Emulgatoren bzw. Dispergatoren kommen in Frage die Gruppe, die gebildet wird von Polyglyceryl-2-dipolyhydroxystearate (Dehymuls®PGPH), Polyglyceryl-3-diisostearate (Lameform®TGI), Polyglyceryl-4-isostearate (Isolan®GI 34), Polyglyceryl-3-oleate, Diisostearyl-Polyglyceryl-3-diisostearate (Isolan®PDI), Polyglyceryl-3-methylglucose distearate (Tego Carey®450), Polyglyceryl-3-beeswax (Cera Bellina®), Polyglyceryl-4-caprate (Polyglycerol caprate T2010/90), Polyglyceryl-3-cetylether (Chimexane®NL), Polyglyceryl-3-distearate (Cremophor®GS 32), Polyglyceryl-2-stesrate (Hostacerin®DGMS) und Polyglyceryl-polyricineoleate (Admul®WOL 1403) sowie deren Gemischen.

Die jeweils einzusetzenden Mengen an kosmetischen oder dermatologischen Hilfsund Trägerstoffen und Parfüm können in Abhängigkeit von der Art des jeweiligen Produktes vom Fachmann durch einfaches Ausprobieren leicht ermittelt werden.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butylund Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Die Lipidphase kann vorteilhaft gewählt werden aus folgender Substanzgruppe:
- Mineralöle, Mineralwachse;
- Öle, wie Triglyceride der Caprin- oder der Caprylsäure, ferner natürlicher Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxan, Diethylpolysiloxan, Diphenylpolysiloxan sowie Mischformen daraus.

Die Ölphasen der Emulsionen, Oleogele bzw. Hydrodispersionen oder Lipodispersionen im Sinne der vorliegenden Erfindung werden vorteilhaft gewählt aus der Gruppe der Ester aus gesättigten und/oder ungesättigten, verzweigten und/oder unverzweig-ten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unver-zweigten Alkoholen einer Kettenlänge von 3 bis 30 C-Atomen. Solche Esteröle können dann vorteilhaft gewählt werden aus der Gruppe Isopropylmyristat, -palmitat, -stearat, -oleat, n-Butylstearat, n-Hexyllaurat, n-Decyloleat, Isooctylstearat, Iso-nonylstearat, Isononylisononanat, 2-Ethylhexylpalmitat, Ethylhexyllaurat, 2-Hexyl-decylstearat, 2-Octyldodecylpalmitat, Oleyloleat, Oleylerucat, Erucyloleat sowie synthetische, halbsynthetische und natürliche Gemische solcher Ester, zB. Jojobaöl.

Ferner kann die Ölphase vorteilhaft gewählt werden aus der Gruppe der verzweigten und unverzweigten Kohlenwasserstoffe und -wachse, der Siliconöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder Siliconölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan),

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft Alkohole, Diole oder Polyole (Niederalkyl), sowie deren Ether, vorzugsweise Ethanol, Isopropanol, Propylenglykol, Glycerin, Ethylenglykol-monoethyloder monobutylether, Propylenglykolmonomethyl, -monoethyl- oder monobutylether, Diethylenglykolmonomethyl- oder -monoethylether und analoge Produkte, ferner Alkohole (Niederalkyl), z.B. Ethanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Eine umfassende Beschreibung der in kosmetischen Mitteln eingesetzten Roh- und Wirkstoffe ist in DE-A 199 19 630 dargestellt.

Es war nicht vorauszusehen, dass die erfindungsgemäßen Indanylidenverbindungen im Vergleich zu den aus der EP-A 823 418 bekannten Verbindungen eine herausragende Auswahl darstellen.

### Beispiele:

### Photostabilität

Im folgenden werden beispielhaft Vergleichsmessungen zwischen den Verbindungen der Kategorie A und den Verbindungen der Kategorie B sowie die Kombination mit anderen marktüblichen UV-Filtern wie OMC (= Octylmethoxycinnamat) oder BMDM (= tert.-Butyl-methoxy-dibenzoylmethan) angeführt. Die Substanzen der Kategorie B zeigen den Fortschritt gegenüber den Substanzen der Kategorie A. Die Bestrahlung wurde in einem Suntester der Firma Heraeus mit einer Bestrahlungsstärke von 765 W/m² (bez. auf Globalsensor) durchgeführt. Die Werte geben die Konzentrations-abnahme der UV-Filter in Prozent nach Bestrahlung (Dosis in J/cm²) wieder.

**Tab.1**

| Formulierung nach Rezepturbeispiel 1 | | | | | |
|---|---|---|---|---|---|
| **Verbindung** | **A1** | **A2** | **B1** | **B2** | **B3** |
| 72 J/cm² | 10% | 1% | 2% | 1% | 1% |
| 144 J/cm² | 13% | 6% | 3% | 2% | 2% |

**Tab.2**

| Formulierung nach Rezepturbeispiel 12 | | | | | | | | | | |
|---|---|---|---|---|---|---|---|---|---|---|
| **Kombination** | **A1** | **OMC** | **A2** | **OMC** | **B1** | **OMC** | **B2** | **OMC** | **B3** | **OMC** |
| 72 J/cm² | 22% | 27% | 14% | 7% | 2% | 12% | 6% | 11% | 6% | 11% |
| 144 J/cm² | 37% | 35% | 26% | 12% | 3% | 19% | 6% | 13% | 6% | 13% |

**Tab.3**

| Formulierung nach Rezeptur | | | | | | | | | |
|---|---|---|---|---|---|---|---|---|---|
| | *Vergleich ohne A oder B* | | *Beispiel 12* | | *Beispiel 4 ohne OMC* | | *Beispiel 4* | | |
| **Kombination** | **OMC** | **BMDM** | **B3** | **OMC** | **B3** | **BMDM** | **B3** | **OMC** | **BMDM** |
| 72 J/cm² | 48% | 68% | 6% | 11% | 2% | 5% | 3% | 17% | 35% |
| 144 J/cm² | 59% | 85% | 6% | 13% | 3% | 13% | 5% | 26% | 54% |

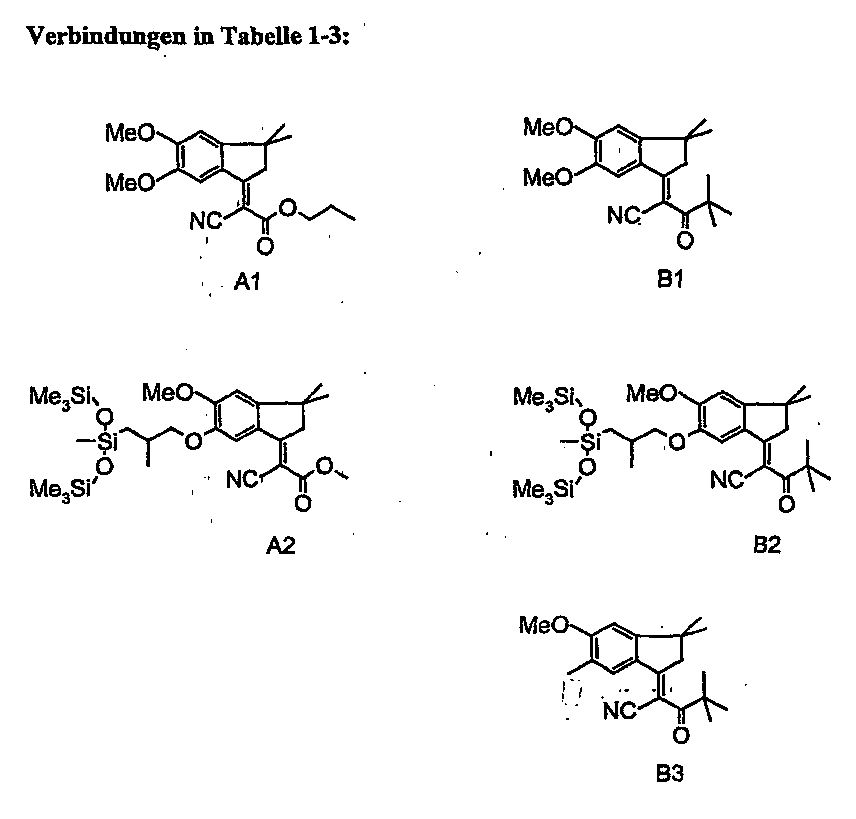

### Beispiel 1

2-(5,6-Dimethoxy-3,3-dimethyl-1-indanyliden)-4,4-dimethyl-3-oxo-pentansäurenitril

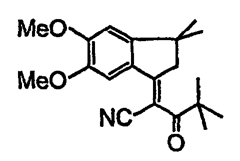

44 g (0,2 mol) 5,6-Dimethoxy-3,3-dimethyl-1-indanon, 25 g (0,2 mol) Pivaloylacetonitril, 32 g Propionsäure und 17 g Ammoniumacetat werden in 80 g Xylol gemischt und auf 120°C 7 Stunden erhitzt. Nach Abkühlen auf Raumtemperatur und Waschen der organischen Phase wird das Xylol abdestilliert und das verbleibende Rohprodukt in Methanol umkristallisiert. Ausbeute: 50 % der Theorie; E^{1/1} 730 (λₘₐₓ 373 nm).

### Beispiel 2

2-(5-Methoxy-3,3,4,6-tetramethyl-1-indanyliden)-4,4-dimethyl-3-oxo-pentansäurenitril

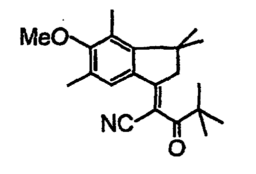

Es wurde analog zu Beispiel 1 ausgehend von 5-Methoxy-3,3,4,6-tetramethyl-1-indanon vorgegangen. Ausbeute: 50 % der Theorie; E^{1/1} 588 (λₘₐₓ 340 nm).

### Beispiel 3

2-(3,3,5,6-tetramethyl-1-indanyliden)-4,4-dimethyl-3-oxo-pentansäurenitril

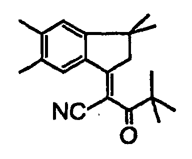

Es wurde analog zu Beispiel 1 ausgehend von 3,3,5,6-tetramethyl-1-indanon vorgegangen. Ausbeute: 55 % der Theorie; E^{1/1} 630 (λₘₐₓ 342 nm).

### Beispiel 4

2-(3,3,6-trimethyl-1-indanyliden)-4,4-dimethyl-3-oxo-pentansäurenitril

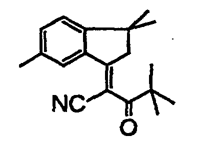

Es wurde analog zu Beispiel 1 ausgehend von 3,3,6-trimethyl-1-indanon vorgegangen. Ausbeute: 45 % der Theorie; E^{1/1} 528/505 (λₘₐₓ 335/316 nm).

### Beispiel 5

2-(5,6-Ethylendioxo-3,3-dimethyl-1-indanyliden)-4,4-dimethyl-3-oxo-pentansäurenitril

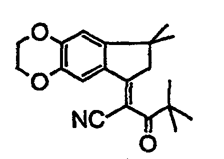

Es wurde analog zu Beispiel 1 ausgehend von 5,6-Ethylendioxo-3,3-dimethyl-1-indanon vorgegangen. Ausbeute: 55 % der Theorie; E^{1/1} 640 (λₘₐₓ 369 nm).

### Beispiel 6

2-(5-Methoxy-3,3,6-trimethyl-1-indanyliden)-4,4-dimethyl-3-oxo-pentansäurenitril

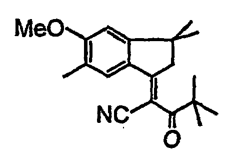

Es wurde analog zu Beispiel 1 ausgehend von 5-Methoxy-3,3,6-trimethyl-1-indanon vorgegangen. Ausbeute: 60 % der Theorie; E^{1/1} 850 (λₘₐₓ 359 nm).

### Beispiel 7

2-[(5-Methoxy-3,3-dimethyl-(2-methyl-3-(1,3,3,3-tetramethyl-1-(trimethyl-silyloxy)-disiloxanyl)-propyl)-indanyliden)]-4,4-dimethyl-3-oxo-pentan-säurenitril 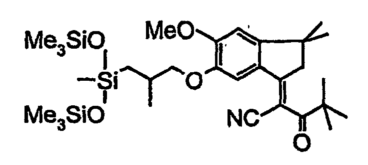
a) 2-(5-Methoxy-3,3-dimethyl-6-hydroxy-1-indanyliden)-4,4-dimethyl-3-oxo-pentan-säurenitril 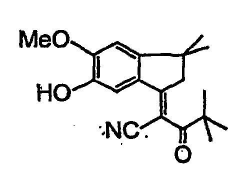 5-Methoxy-3,3-dimethyl-6-hydroxy-1-indanon wird nach dem Beispiel 1 umgesetzt. Ausbeute: 50% d.Th.
b) 2-(5-Methoxy-3,3-dimethyl-6-(2-methyl-propenyloxy)-1-indanyliden)-4,4-dimethyl-3-oxo-pentansäurenitril

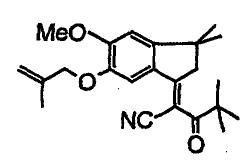

136 g (0,43 mol) der Verbindung unter a) werden zusammen mit 95 g Kaliumcarbonat in 470 g N-Methylpyrrolidinon gegeben, auf 70°C erhitzt und innerhalb von 30 min mit 42 g (0,46 mol) Methallylchlorid versetzt. Man erhitzt noch 3 h bei 70°C, kühlt ab auf Raumtemperatur und extrahiert das Produkt mit Essigester. Ausbeute: 45% d.Th.

90g (130 mmol) der Verbindung unter b), 29g (130 mmol) 1,1,1,3,5,5,5-Heptamethyltrisiloxan werden in Gegenwart katalytischer Mengen Divinyl-tetramethyl-Platin-Komplex in 90g Toluol und Stickstoffatmosphäre 20h auf 80°C gehalten. Nach Abdestillieren des Lösungsmittels wird der Rückstand über ein Kugelrohr destilliert. Man erhält 50g (70% d. Th.) des gewünschten Produktes als gelbes Öl; E^{1/1} 400 (λₘₐₓ 373 nm).

### Beispiel 8

2-(6-Acetoxy-3,3-dimethyl-5-methoxy-1-indanyliden)-4,4-dimethyl-3-oxo-pentansäurenitril

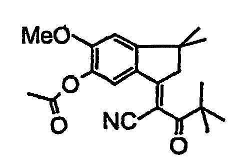

54 g (0,17 mol) der Verbindung unter a) werden mit 13 g (0,17 mol) Acetylchlorid in N-Methylpyrrolidinon bei 40°C innerhalb 5h umgesetzt. Ausbeute: 98 % d.Th. E^{1/1} 420/280 (λₘₐₓ 355/302 nm).

### Beispiel 9

2-(3,3-Dimethyl-5-tert.-butyl-1-indanyliden)-3-(1'-methylcyclohexyl)-3-oxopropionsäurenitril

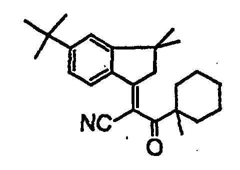

3,3-Dimethyl-5-tert.-butyl-1-indanon werden mit 3-(1'-Methylcyclohexyl)-3-oxopropionsäurenitril analog zu Beispiel 1 umgesetzt.
Ausbeute: 40 % d.Th.
E^{1/1} 580 (λₘₐₓ 355 nm).

### Beispiel 10

2-(3,3,5-Trimethyl-1-indanyliden)-3-phenyl-3-oxo-propionsäurenitril

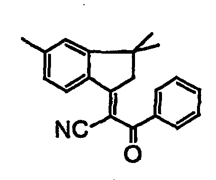

3,3,5-Trimethyl-1-indanon werden mit Benzoylacetonitril analog zu Beispiel 1 umgesetzt.
Ausbeute: 50 % d.Th.
E^{1/1} 600 (λₘₐₓ 350 nm).

### Rezepturbeispiel 1

Sonnenschutz Soft Creme (O/W), in-vitro SPF 3, wasserfest

| Teil | Rohstoffe | INCI Bezeichnung | % (Gew.) |
|---|---|---|---|
| A | Crodafos MCA | Cetyl Phosphate | 1.50 |
| | Cutina MD | Glyceryl Stearate | 2.00 |
| | Copherol 1250 | Tocopherylacetate | 0.50 |
| | Lanette 16 | Cetyl Alcohol | 1.00 |
| | Tegosoft TN | C 12-15 Alkyl Benzoate | 24.00 |
| | Prisorine 3505 | Isostearic Acid | 1.00 |
| | UV-Absorber gemäß Formel I | | 3.00 |
| | | | |
| B | Wasser, dest. | Water (Aqua) | 59.60 |
| | EDETA B fl. | Tetrasodium EDTA | 0.20 |
| | Glycerin, 99% | Glycerin | 3.00 |
| | Phenoxyethanol | Phenoxyethanol | 0.70 |
| | Solbrol M | Methylparaben | 0.20 |
| | Solbrol P | Propylparaben | 0.10 |
| | Carbopol ETD 2050 | Carbomer | 0.20 |
| | | | |
| C | Natronlauge, 10 % aq. | Sodium Hydroxide | 2.70 |
| | | | |
| D | Parfümöl | Parfum (Fragrance) | 0.30 |

### Herstellungsverfahren

- Teil A:: Auf ca. 85°C erhitzen.
- Teil B:: Rohstoffe ohne Carbopol einwiegen. Carbopol mit Ultra Turrax eindispergieren. Auf ca. 85°C erhitzen. B zu A geben.
- Teil C:: Sofort zu A/B geben und anschließend heiß homogenisieren (Ultra Turrax). Unter Rühren abkühlen lassen. '
- , Teil D:: Zugeben und verrühren.

| | |
|---|---|
| in-vitro SPF (Labsphere Ultraviolet Transmittance Analyzer) | 3 |
| Boots Star Rating | 4 |
| Broad Spectrum Rating (Diffey) | 4 |
| Kritische Wellenlänge (90% Absorption bei λ in nm) | 385 |
| UV-Filter Substantivität nach Wässerung | 97 % |

### Rezepturbeispiel 2

Sonnenschutzlotion (O/W), in-vitro SPF 20

| Teil | Rohstoffe | INCI Bezeichnung | % (Gew.) |
|---|---|---|---|
| A | Crodafos MCA | Cetyl Phosphate | 1.50 |
| | Cutina MD | Glyceryl Stearate | 2.00 |
| | Copherol 1250 | Tocopherylacetate | 0.50 |
| | Lanette 16 | Cetyl Alcohol | 1.00 |
| | Tegosoft TN | C 12-15 Alkyl Benzoate | 10.60 |
| | Prisorine 3505 | Isostearic Acid | 1.00 |
| | UV-Absorber gemäß Formel I | | 2.00 |
| | Neo Heliopan® AV | Ethyl Hexyl Methoxycinnamate | 5.00 |
| | | | |
| B | Wasser, dest. | Water (Aqua) | 55.07 |
| | EDETA B fl. | Tetrasodium EDTA | 0.20 |
| | Glycerin, 99% | Glycerin | 3.00 |
| | Phenoxyethanol | Phenoxyethanol | 0.70 |
| | Solbrol M | Methylparaben | 0.20 |
| | Solbrol P | Propylparaben | 0.10 |
| | Carbopol ETD 2050 | Carbomer | 0.20 |
| | | | |
| C | Natronlauge, 10 % aq. | Sodium Hydroxide | 3.30 |
| | Neo Heliopan® Hydro, 15% ige Lösung neutralisiert mit NaOH | Phenylbenzimidazole Sulfonic Acid | 13.33 |
| | | | |
| D | Parfümöl | Parfum (Fragrance) | 0.30 |

### Herstellungsverfahren

- Teil A:: Auf ca. 85°C erhitzen.
- Teil B:: Rohstoffe ohne Carbopol einwiegen. Carbopol mit Ultra Turrax eindispergieren. Auf ca. 85°C erhitzen. B zu A geben.
- Teil C:: Sofort zu A/B geben und anschließend heiß homogenisieren (Ultra Turrax). Unter Rühren abkühlen lassen.
- Teil D:: Zugeben und verrühren.

| | |
|---|---|
| in-vitro SPF (Labsphere Ultraviolett Transmittance Analyzer) | 20 |
| Boots Star Rating | 2 |
| Broad Spectrum Rating (Diffey) | 4 |
| Kritisehe Wellenlänge (90% Absorption bei λ in nm) | 378 |

### Rezepturbeispiel 3

Sonnenschutzmilch (O/W), in-vitro SPF 6

| Teil | Rohstoffe | INCI Bezeichnung | % (Gew.) |
|---|---|---|---|
| A | Tegin M | Glyceryl Stearate | 2.50 |
| | Tagat S | PEG-30 Glyceryl Stearate | 1.95 |
| | Lanette O | Cetearyl Alcohol | 2.20 |
| | Copherol 1250 | Tocopherylacetat | 0.50 |
| | Miglyol 8810 | Butylene Glycol Dicaprylate/ Caprate | 12.00 |
| | Tegosoft TN | C12 - C15 Alkylbenzoate | 8.00 |
| | Phenonip | Phenoxyethanol (and) Methylparaben (and) Butylparaben (and) Ethyl-paraben (and) Propylparaben | 0.15 |
| | UV-Absorber gemäß Formel I | | 5.00 |
| B | Wasser, dest. | Water (Aqua) | 43.90 |
| | EDETABD | Disodium EDETA | 0.10 |
| | 1,2-Propylenglykol | Propylene Glycol | 2.00 |
| | Phenonip | Phenoxyethanol (and) Methylparaben (and) Butylparaben (and) Ethylparaben (and) Propylparaben | 0.30 |
| | | | |
| C | Wasser, dest. | Water (Aqua) | 19.00 |
| | Carbopol 2050 | Carbomer | 0.40 |
| | NaOH, 10%ig | Sodium Hydroxide | 1.70 |
| | | | |
| D | Parfümöl | Parfum (Fragrance) | 0.30 |

### Herstellungsverfahren

- Teil: A: Auf 80-85°C erhitzen.
- Teil B:: Auf 80-85°C erhitzen, unter Rühren Teil B zu Teil A geben.
- Teil C:: Carbopol in das Wasser eindispergieren und unter Rühren mit NaOH neutralisieren.
- Teil C: bei ca. 60°C unter Rühren zugeben. Auf RT abkühlen lassen.
- Teil D:: Zugeben und verrühren.

| | |
|---|---|
| in-vitro SPF (Labsphere Ultraviolet Transmittance Analyzer) | 6 |
| Boots Star Rating | 4 |
| Broad Spectrum Rating (Diffey) | 4 |
| Kritische Wellenlänge (90% Absorption bei λ in nm) | 385 |

### Rezepturbeispiel 4

Sonnenschutzlotion (O/W), in-vitro SPF 21

| Teil | Rohstoffe | INCI Bezeichnung | % (Gew.) |
|---|---|---|---|
| A | Tegin M | Glyceryl Stearate | 2.50 |
| | Tagat S | PEG-30 Glyceryl Stearate | 1.95 |
| | Lanette O | Cetearyl Alcohol | 2.20 |
| | Copherol 1250 | Tocopherylacetat | 0.50 |
| | Miglyol 8810 | Butylene Glycol Dicaprylate / Caprate | 12.00 |
| | Tegosoft TN | C12 - C15 Alkylbenzoate | 8.00 |
| | Phenonip | Phenoxyethanol (and) Methylparaben (and) Butylparaben (and) Ethylparaben (and) Propylparaben | 0.20 |
| | UV-Absorber gemäß Formel I | | 2.00 |
| | Neo Heliopan® AV | Ethylhexyl Methoxycinnamate | 5.00 |
| | Neo Heliopan® 357 | Butyl Methoxydibenzoylmethane | 1.00 |
| B | Wasser, dest. | Water (Aqua) | 39.35 |
| | EDETA BD | Disodium EDETA | 0.10 |
| | 1,2-Propylenglykol | Propylene Glycol | 2.00 |
| | Phenonip | Phenoxyethanol (and) Methylparaben (and) Butylparaben (and) Ethylparaben (and) Propylparaben | 0.30 |
| | Vitamin C | Ascorbic Acid | 0.10 |
| | | | |
| C | Wasser, dest. | Water (Aqua) | 20.00 |
| | Carbopol 2050 | Carbomer | 0.40 |
| | NaOH, 10%ig | Sodium Hydroxide | 1.70 |
| | | | |
| D | Parfümöl | Parfum (Fragrance) | 0.30 |

### Herstellungsverfahren

Teil A:Auf 80-85°C erhitzen.
Teil B: Auf 80-85°C erhitzen, unter Rühren Teil B zu Teil A geben.
Teil C: Carbopol in das Wasser eindispergieren und unter Rühren mit NaOH neutralisieren.
Teil C bei ca. 60°C unter Rühren zugeben. Auf RT abkühlen lassen.
Teil D: Zugeben und verrühren.

| | |
|---|---|
| in-vitro SPF (Labsphere Ultraviolet Transmittance Analyzer) | 21 |
| Boots Star Rating | 3 |
| Broad Spectrum Rating (Diffey) | 4 |
| Kritische Wellenlänge (90% Absorption bei λ in nm) | 379 |

### Rezepturbeispiel 5

Sonnenschutzlotion (O/W), in-vitro SPF 11

| Teil | Rohstoffe | INCI Bezeichnung | % (Gew.) |
|---|---|---|---|
| A | Eumulgin VL 75 | Lauryl Glucoside (and) Polyglyceryl-2-Dipolyhydroxystearate (and) Glycerin | 3.00 |
| | Tegosoft TN | C12-25 Alkyl Benzoate | 20.00 |
| | Copherol 1250 | Tocopherylacetat | 0.50 |
| | UV-Absorber gemäß Formel I | | 3.00 |
| | Parfümöl | Parfum (Fragrance) | 0.20 |
| | Neo Heliopan® 303 | Octocrylene | 5.00 |
| | Carbopol 2984 | Carbomer | 0.35 |
| | Pemulen TR-1 | Acrylates/C10-30 Alkylacrylate Crosspolymer | 0.15 |
| | | | |
| B | Wasser, dest. | Water (Aqua) | 60.50 |
| | EDETABD | Disodium EDTA | 0.10 |
| | Glycerin, 99% | Glycerin | 5.00 |
| | Phenoxyethanol | Phenoxyethanol | 0.70 |
| | Solbrol M | Methylparaben | 0.20 |
| | Solbrol P | Propylparaben | 0.10 |
| | | | |
| C | NaOH, 10%ig | Sodium Hydroxide | 1.20 |

### Herstellungsverfahren

- Teil A:: UV-Absorber gemäß Formel 1 in den Ölen bzw flüssigen UV-Filtern lösen (Erwärmen bis ca. 70°C). Abkühlen lassen auf ca. 30°C, restliche Bestandteile außer Carbopol und Pemulen zufügen und bei Raumtemperatur mischen (ca. 5 Minuten rühren). Carbopol und Pemulen einrühren.
- Teil B:: Solbrole unter Erwärmen in Phenoxyethanol lösen. Mit Wasser und Glycerin mischen, unter Rühren zu Teil A geben. Ca. 60 Minuten rühren.
- Teil C:: Zu A/B geben, mit dem Ultra Turrax homogenisieren.

| | |
|---|---|
| in-vitro SPF (Labsphere Ultraviolet Transmittance Analyzer) | 11 |
| Boots Star Rating | 4 |
| Broad Spectrum Rating (Diffey) | 4 |
| Kritische Wellenlänge (90% Absorption bei λ in nm) | 382 |

### Rezepturbeispiel 6

Sonnenschutzcreme (W/O), in-vitro SPF 4, wasserfest

| Teil | Rohstoffe | INCI Bezeichnung | % (Gew.) |
|---|---|---|---|
| A | Dehymuls PGPH | Polyglyceryl-2 Dipolyhydroxystearate | 5.00 |
| | Copherol 1250 | Tocopherylacetat | 0.5 0 |
| | Permulgin 3220 | Ozokerite | 0.50 |
| | Zinkstearat | Zinc Stearate | 0.50 |
| | Tegosoft TN | C12-15 Alkyl Benzoate | 25.00 |
| | UV-Absorber gemäß Formel I | | 5.00 |
| | | | |
| B | Wasser, dest. | Water (Aqua) | 57.90 |
| | EDETA BD | Disodium EDTA | 0.10 |
| | Glycerin, 99% | Glycerin | 4.00 |
| | Phenoxyethanol | Phenoxyethanol | 0.70 |
| | Solbrol M | Methylparaben | 0.20 |
| | Solbrol P | Propylparaben | 0.10 |
| | Magnesiumsulfat | Magnesium Sulfate | 0.50 |

### Herstellungsverfahren

- Teil A:: Aufca. 85°C erhitzen.
- Teil B:: Auf ca. 85°C erhitzen (ohne Zinkoxid; Zinkoxid mit dem Ultra Turrax eindispergieren).
B zu A geben.
Unter Rühren abkühlen lassen, anschließend homogenisieren.

| | |
|---|---|
| in-vitro SPF (Labsphere Ultraviolet Transmittance Analyzer) | 4 |
| Boots Star Rating | 4 |
| Broad Spectrum Rating (Diffey) | 4 |
| Kritische Wellenlänge (90% Absorption bei λ in nm) | 384 |
| UV-Filter Substantivität nach Wässerung | 95 % |

### Rezepturbeispiel 7

Sonnenschutz Softcreme (W/O) , in-vitro SPF 40

| Teil | Rohstoffe | INCI Bezeichnung | % (Gew.) |
|---|---|---|---|
| A | Dehymuls PGPH | Polyglyceryl-2 Dipolyhydroxystearate | 5.00 |
| | Copherol 1250 | Tocopherylacetat | 0.50 |
| | Permulgin 3220 | Ozokerite | 0.50 |
| | Zinkstearat | Zinc Stearate | 0.50 |
| | Tegosoft TN | C12-15 Alkyl Benzoate | 10.00 |
| | UV-Absorber gemäß Formel I | | 2.00 |
| | Neo Heliopan® 303 | Octocrylene | 5.00 |
| | Neo Heliopan® MBC | 4-Methylbenzylidene Camphor | 3.00 |
| | Zinkoxid neutral H&R | Zinc Oxide | 5.00 |
| | | | |
| B | Wasser, dest. | Water (Aqua) | 62.90 |
| | EDETA BD | Disodium EDTA | 0.10 |
| | Glycerin, 99% | Glycerin | 4.00 |
| | Phenoxyethanol | Phenoxyethanol | 0.70 |
| | Solbrol M | Methylparaben | 0.20 |
| | Solbrol P | Propylparaben | 0.10 |
| | Magnesiumsulfat | Magnesium Sulfate | 0.50 |
| | | | |
| C | Parfümöl | Parfum (Fragrance) | 0.20 |

### Herstellungsverfahren

- Teil A:: Auf ca. 85°C erhitzen.
- Teil B:: Auf ca. 85°C erhitzen (ohne Zinkoxid; Zinkoxid mit dem Ultra Turrax eindispergieren).
B zu A geben.
Unter Rühren abkühlen lassen.
- Teil C:: Zugeben und anschließend homogenisieren

| | |
|---|---|
| in-vitro SPF (Labsphere Ultraviolet Transmittance Analyzer) | 40 |
| Boots Star Rating | 3 |
| Broad Spectrum Rating (Diffey) | 4 |
| Kritische Wellenlänge (90% Absorption bei λ in nm) | 379 |

### Rezepturbeispiel 8

Sonnenschutzmilch (W/O)

| Teil | Rohstoffe | INCI Bezeichnung | % (Gew.) |
|---|---|---|---|
| A | Dehymuls PGPH | Polyglyceryl-2 Dipolyhydroxystearate | 3.00 |
| | Bienenwachs 8100 | Beeswax | 1.00 |
| | Monomuls 90-0-18 | Glyceryl Oleate | 1.00 |
| | Zinkstearat | Zinc stearate | 1.00 |
| | Cetiol SN | Cetearyl Isononanoate | 5.00 |
| | Cetiol OE | Dicaprylyl Ether | 5.00 |
| | Tegosoft TN | C12-15 Alkyl Benzoate | 4.00 |
| | Copherol 1250 | Tocopherylacetat | 0.50 |
| | Solbrol P | Propylparaben | 0.10 |
| | Neo Heliopan® OS | Ethylhexyl Salicylate | 5.00 |
| | Neo Heliopan® AV | Ethylhexyl Methoxycinnamate | 7.50 |
| | UV-Absorber gemäß Formel I | | 1.50 |
| | | | |
| B | Wasser, dest. | Water (Aqua) | 44.10 |
| | Trilon BD | Disodium EDTA | 0.10 |
| | Glycerin, 99% | Glycerin | 5.00 |
| | Solbrol M | Methylparaben | 0.20 |
| | Phenoxyethanol | Phenoxyethanol | 0.70 |
| | Neo Heliopan® AP 10%ige Lösung neutralisiert mit NaOH | Disodium Phenyl Dibenzimidazole Tetrasulfonate | 15.00 |
| | | | |
| C | Parfümöl | Parfum (Fragrance) | 0.30 |
| | Bisabolol | Bisabolol | 0.10 |

### Herstellungsverfahren

- Teil A:: Auf ca. 85°C erhitzen.
- Teil B:: Auf ca. 85°C erhitzen. B zu A geben.Unter Rühren abkühlen lassen.
- Teil C:: Zugeben und anschließend homogenisieren.

### Rezepturbeispiel 9

Tagespflegecreme mit UV-Schutz

| Teil | Rohstoffe | INCI Bezeichnung | % (Gew.) |
|---|---|---|---|
| A | Emulgade PL 68/50 | Cetearyl Glycoside (and) Cetearyl Alcohol | 4.50 |
| | Cetiol PGL | Hexyldecanol (and) Hexyldecyl Laurate | 8.00 |
| | Myritol 331 | Cocoglycerides | 8.00 |
| | Copherol 1250 | Tocopheryl Acetat | 0.50 |
| | Neo Heliopan® E1000 | Isoamyl-p- Methoxycinnamate | 2.00 |
| | UV-Absorber gemäß Formel I | | 2.00 |
| | | | |
| B | Wasser, dest | Water (Aqua) | 45.40 |
| | Glycerin | Glycerin | 3.00 |
| | Phenonip | Phenoxyethanol (and) Methylparaben (and) Butyparaben (and) Ethyparaben (and) Propylparaben | 0.50 |
| | | | |
| C | Wasser, dest | Water (Aqua) | 25.00 |
| | Carbopol ETD 2050 | Carbomer | 0.20 |
| | NaOH, 10%ig | Sodium Hydroxide | 0.60 |
| | | | |
| D | Parfümöl | Parfum (Fragrance) | 0.30 |

### Herstellungsverfahren

- Teil A:: Auf 80°C erhitzen.
- Teil B:: Auf 80°C erhitzen.Unter Rühren zu Teil A geben.
- Teil C:: Carbopol in Wasser dispergieren und mit Natronlauge neutralisieren. Bei ca. 55°C zu TeilA/Bgeben.
- Teil D:: Bei RT zugeben und homogenisieren.

### Rezepturbeispiel 10

Sonnenschutzspray

| Teil | Rohstoffe | INCI Bezeichnung | % (Gew.) |
|---|---|---|---|
| A | Wasser, dem. | Water (Aqua) | 69.50 |
| | Glycerin, 99% | Glycerin | 4.00 |
| | 1,3-Butylenglykol | Butylene Glycol | 5.00 |
| | D-Panthenol | Panthenol | 0.50 |
| | Lara Care A-200 | Galactoarabinan | 0.25 |
| | | | |
| B | Baysiloneöl M 10 | Dimethicone | 1.00 |
| | Edeta BD | Disodium EDTA | 0.10 |
| | Copherol 1250 | Tocopheryl Acetate | 0.50 |
| | Cetiol OE | Dicaprylyl Ether | 3.00 |
| | Neo Heliopan® HMS | Homosalate | 5.00 |
| | Neo Heliopan® AV | Ethylhexyl Methoxycinnamate | 6.00 |
| | Neo Heliopan® 357 | Butyl Methoxydibenzoylmethane | 1.00 |
| | UV-Absorber gemäß Formel I | | 2.00 |
| | Alpha Bisabolol nat. H&R | Bisabolol | 0.10 |
| | Pemulen TR-2 | Acrylates/C10-30 Alkyl Acrylate Crosspolymer | 0.25 |
| | | | |
| C | Phenoxyethanol | Phenoxyethanol | 0.70 |
| | Solbrol M | Methylparaben | 0.20 |
| | Solbrol P | Propylparaben | 0.10 |
| | | | |
| D | NaOH, 10 %ig | Sodium Hydroxide | 0.60 |
| | | | |
| E | Parfümöl | Fragrance (Parfum) | 0.20 |

### Herstellungsverfahren

- Teil A:: Lara Care A-200 unter Rühren in den anderen Bestandteilen von Teil A lösen.
- Teil B:: Alle Rohstoffe (ohne Pemulen) einwiegen und die kristallinen Substanzen unter Erwärmen lösen. Pemulen eindispergieren. Teil B zu Teil A geben und 1 Minute homogenisieren.
- Teil C+D: zugeben und nochmals 1-2 Minuten mit dem Ultra Turrax homogenisieren.

### Rezepturbeispiel 11

Sonnenschutz Hydrodispersionsgel (Balm)

| Teil | Rohstoffe | INCI Bezeichnung | % (Gew.) |
|---|---|---|---|
| A | Wasser, dest. | Water (Aqua) | 74.90 |
| | Carbopol 1342 | Acrylates/C10-30 Alkyl Acrylate Crosspolomer | 1.00 |
| | Triethanolamine | Triethanolamine | 1.20 |
| | | | |
| B | Neo Heliopan® Hydro, 30%ige Lösung neutralisiert mit TEA | Phenylbenzimidazole Sulfonic Acid | 10.00 |
| | | | |
| C | Neo Heliopan® AV | Ethylhexyl Methoxycinnamate | 3.00 |
| | UV-Absorber gemäß Formel I | | 2.00 |
| | Isopmpylmyristat | Isopropyl Myristate | 4.00 |
| | Baysilone ÖL PK 20 | Phenyl Trimethicone | 3.00 |
| | Phenonip | Phenoxyethanol (and) Methylparaben (and) Butyparaben (and) Ethyparaben (and) Propylparaben | 0.50 |
| | Parfümöl | Parfum (Fragrance) | 0.30 |
| | Bisabolol nat H&R | Bisabolol | 0.10 |

### Herstellungsverfahren

- Teil A:: Carbopol in Wasser dispergieren und mit Natronlauge neutralisieren.
- Teil B:: Unter Rühren zu Teil A geben.
- Teil C:: Kristalline Bestandteile unter Erwärmen (max.40°C) in den anderen Rohstoffen von Teil C lösen und zu Teil A/B geben. Gut verrühren und anschließend homogenisieren (Homozenta)

### Rezepturbeispiel 12

Hair Conditioner mit UV-Filtern

| Teil | Rohstoffe | INCI Bezeichnung | % (Gew.) |
|---|---|---|---|
| A | Emulgade 1000 NI | Cetearyl Alcohol (and) Ceteareth-20 | 2.00 |
| | Lanette 16 | Cetyl Alcohol | 1.00 |
| | Neo Heliopan® AV | 2 -Ethylhexyl Methoxycinnamate | 3.00 |
| | UV-Absorber gemäß Formel I | ' | 1.00 |
| | | | |
| B | Wasser, dest | Water(Aqua) | 91.70 |
| | Edeta BD | Disodium EDTA | 0.10 |
| | Phenonip | Phenoxyethanol (and) Methylparaben (and) Butyparaben (and) Ethyparaben (and) Propylparaben | 0.40 |
| | Dehyquart A-CA | Cetrimonium Chloride | 0.20 |
| | NaOH, 1%ig | Sodium Hydroxide | 0.30 |
| | | | |
| C | Parfümöl | Parfum (Fragrance) | 0.30 |

### Herstellungsverfahren

- Teil A:: Auf 80°C erhitzen.
- Teil B:: Auf 80°C erhitzen. Unter Rühren zu Teil A geben.
- Teil C:: Bei 40°C zugeben und aufRT abkühlen.

### Rezepturbeispiel 13

Sonnenschutzlotion (O/W)

| Teil | Rohstoffe | INCI Bezeichnung | % (Gew.) |
|---|---|---|---|
| A | Tegin M | Glyceryl Stearate | 2.50 |
| | Tagat S | PEG-30 Glyceryl Stearate | 1.95 |
| | Lanette O | Cetearyl Alcohol | 2.20 |
| | Hallbrite TQ | Diethylhexylnaphthalate | 7,00 |
| | Cetiol B | Dibutyl Adipate | 5,00 |
| | Tegosoft TN | C12 - C15 Alkylbenzoate | 4.00 |
| | Myritol PC | Propylene Glycol Dicaprylate/Dicaprate | 4.00 |
| | Phenonip | Phenoxyethanol (and) Methylparaben (and) Butylparaben (and) Ethyl-paraben (and) Propylparaben | 0.15 |
| | UV-Absorber gemäß Formel I | | 2.00 |
| | Neo Heliopan® AV | Ethylhexyl Methoxycinnamate | 5.00 |
| | | | |
| B | Wasser, dest. | Water (Aqua) | 42,80 |
| | 1,2-Propylenglykol | Propylene Glycol | 2.00 |
| | Phenonip | Phenoxyethanol (and) Methylparaben (and) Butylparaben (and) Ethylparaben (and) Propylparaben | 0.30 |
| | | | |
| C | Wasser, dest. | Water (Aqua) | 19.00 |
| | Carbopol 2050 | Carbomer | 0.40 |
| | NaOH, 10%ig | SodiumHydroxide | 1.70 |

### Herstellungsverfahren

- Teil A:: Auf 80-85°C erhitzen.
- Teil B:: Auf 80-85°C erhitzen, unter Rühren Teil B zu Teil A geben.
- Teil C:: Carbopol in das Wasser eindispergieren und unter Rühren mit NaOH neutralisieren. Teil C bei ca. 60°C unter Rühren zugeben.

## Patentansprüche

1. Verfahren zur Herstellung von Indanylidenverbindungen der Formel 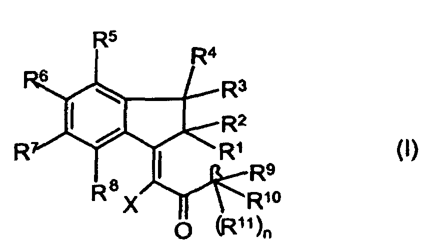 worin
R¹ bis R⁴ unabhängig voneinander Wasserstoff, C₁-C₂₀-Alkyl oder C₅-C₁₀-Cycloalkyl bedeuten, mit der Maßgabe, dass auch zwei Substituenten an benachbarten C-Atomen zusammen eine gegebenenfalls substituierte C₁-C₄-Alkylengruppe bedeuten können;
weiterhin unabhängig voneinander C₂-C₂₀-Alkyl, in der mindestens eine Methylen-gruppe durch Sauerstoff, C₃-C₂₀-Alkenyl, C₃-C₂₀-Alkinyl oder einer Gruppe S ersetzt sein kann, bedeuten können,
wobei S eine Silan-, eine Oligosiloxan- oder eine Polysiloxan-Gruppe sein kann;
R⁵ bis R⁸ unabhängig voneinander Wasserstoff, C₁-C₂₀-Alkyl oder C₅-C₁₀-Cycloalkyl oder C₁ -C₂₀-Alkoxy, C₅-C₁₀-Cycloalkoxy, Hydroxy, Acetoxy, Acetamino, Carboxy, Carbalkoxy oder Carbamoyl bedeuten,
zusätzlich zwei Substituenten von R⁵ bis R⁸ an benachbarten C-Atomen zusammen einen 5-7-gliedrigen Ring bilden können, der bis zu drei Heteroatome, insbesondere Sauerstoff oder Stickstoff enthält, wobei die Ringatome durch exocyclisch doppelt gebundenen Sauerstoff (Ketogruppe) substituiert sein können,
weiterhin im Fall von Alkoxy unabhängig voneinander C₂-C₂₀-Alkyl, in der mindestens eine Methylengruppe durch Sauerstoff, C₃-C₂₀-Alkenyl, C₃-C₂₀ Alkinyl oder einer Gruppe S ersetzt sein kann, bedeuten können,
wobei S eine Silan-, eine Oligosiloxan- oder eine Polysiloxan-Gruppe sein kann;
X für Cyano, CON(R)₂ oder CO₂R, wobei R für Wasserstoff oder C,-C₈-Alkyl steht;
n für 1 oder 0 steht;
R⁹ bis R¹¹ im Fall von n=1 Wasserstoff, C₁-C₂₀-Alkyl oder C₅-C₁₀-Cycloalkyl, Aryl oder Heteroaryl bedeuten können,
zusätzlich zwei Substituenten von R⁹ bis R¹¹ gemeinsam mit dem β-Atom einen 3-7-gliedrigen Ring, der bis zu drei Heteroatome, insbesondere Sauerstoff oder Stickstoff enthalten kann, bilden können,
weiterhin im Fall von n=0 R⁹ und R¹⁰ gemeinsam mit dem β-Atom einen Aryl oder Heteroaryl-Rest bedeuten
und bei dem Indanylidenverbindungen der Formel (I) durch (Knoevenagel)-Kondensation von Verbindungen der Formel 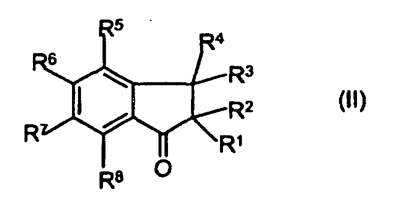 wobei R¹ bis R⁸ die oben genannte Bedeutung haben,
mit Verbindungen der Formel 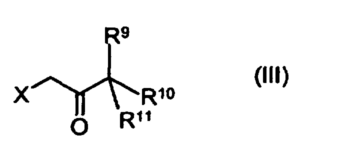 wobei R⁹ bis R¹¹ und X die oben genannte Bedeutung haben, erhalten werden.

2. Indanylidenverbindungen der Formel

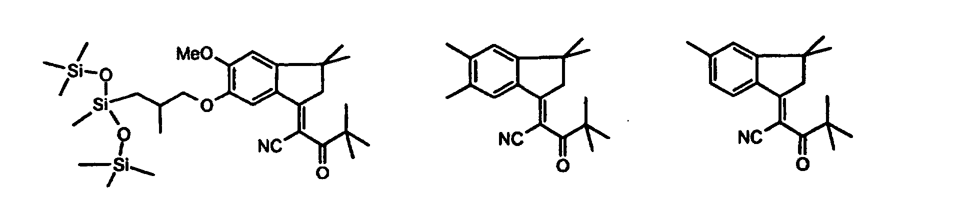

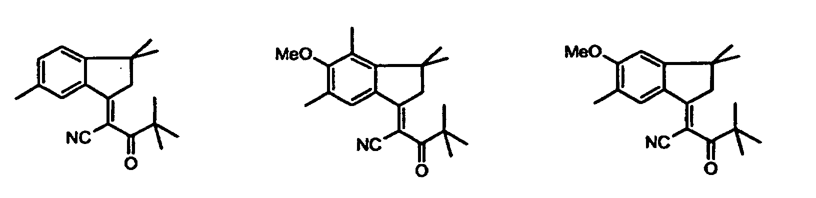

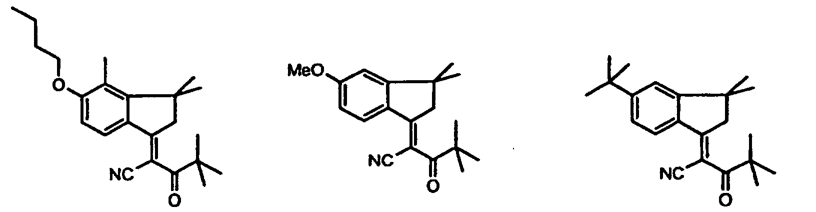

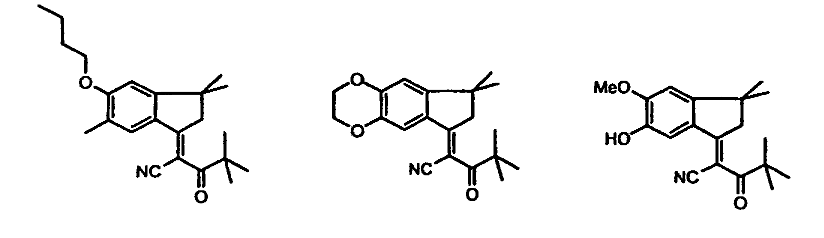

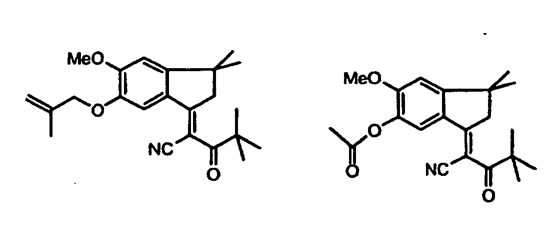

3. Kosmetische Mittel zum Schutz von Haut und Haaren enthaltend Indanylidenverbindungen der Formel 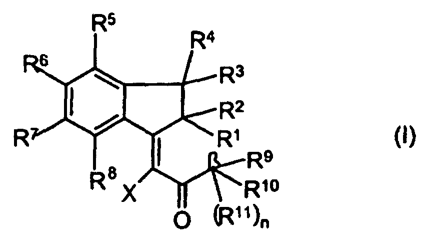 worin
R¹ bis R⁴ unabhängig voneinander Wasserstoff, C₁-C₂₀-Alkyl oder C₅-C₁₀-Cycloalkyl bedeuten, mit der Maßgabe, dass auch zwei Substituenten an benachbarten C-Atomen zusammen eine gegebenenfalls substituierte C₁-C₄-Alkylengruppe bedeuten können;
weiterhin unabhängig voneinander C₂-C₂₀-Alkyl, in der mindestens eine Methylen-gruppe durch Sauerstoff, C₃-C₂₀-Alkenyl, C₃-C₂₀-Alkinyl oder einer Gruppe S ersetzt sein kann, bedeuten können,
wobei S eine Silan-, eine Oligosiloxan- oder eine Polysiloxan-Gruppe sein kann;
R⁵ bis R⁸ unabhängig voneinander Wasserstoff, C₁-C₂₀-Alkyl oder C₅-C₁₀-Cycloalkyl oder C₁-C₂₀-Alkoxy, C₅-C₁₀-Cycloalkoxy, Hydroxy, Acetoxy, Acetamino, Carboxy, Carbalkoxy oder Carbamoyl bedeuten,
zusätzlich zwei Substituenten von R⁵ bis R⁸ an benachbarten C-Atomen zusammen einen 5-7-gliedrigen Ring bilden können, der bis zu drei Heteroatome, insbesondere Sauerstoff oder Stickstoff enthält, wobei die Ringatome durch exocyclisch doppelt gebundenen Sauerstoff (Ketogruppe) substituiert sein können,
weiterhin im Fall von Alkoxy unabhängig voneinander C₂-C₂₀-Alkyl, in der mindestens eine Methylengruppe durch Sauerstoff, C₃-C₂₀-Alkenyl, C₃-C₂₀ Alkinyl oder einer Gruppe S ersetzt sein kann, bedeuten können,
wobei S eine Silan-, eine Oligosiloxan- oder eine Polysiloxan-Gruppe sein kann;
X für Cyano, CON(R)₂ oder CO₂R wobei R für Wasserstoff oder C₁-C₈-Alkyl steht;
n für 1 oder 0 steht;
R⁹ bis R¹¹ im Fall von n=1 Wasserstoff, C₁-C₂₀-Alkyl oder C₅-C₁₀-Cycloalkyl, Aryl oder Heteroaryl bedeuten können,
zusätzlich zwei Substituenten von R⁹ bis R¹¹ gemeinsam mit dem β-Atom einen 3-7-gliedrigen Ring, der bis zu drei Heteroatome, insbesondere Sauerstoff oder Stickstoff enthalten kann, bilden können,
weiterhin im Fall von n=0 R⁹ und R¹⁰ gemeinsam mit dem β-Atom einen Aryl oder Heteroaryl-Rest bedeuten.

4. Kosmetische Mittel zum Schutz von Haut und Haaren nach Anspruch 3, enthaltend

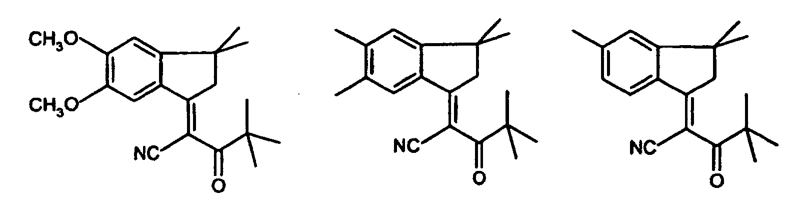

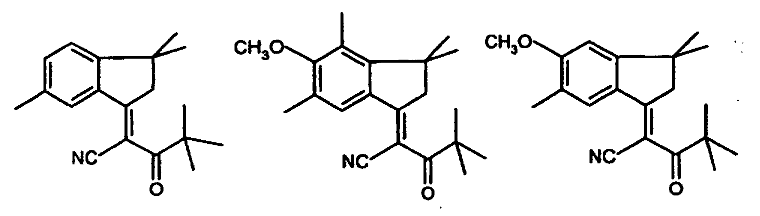

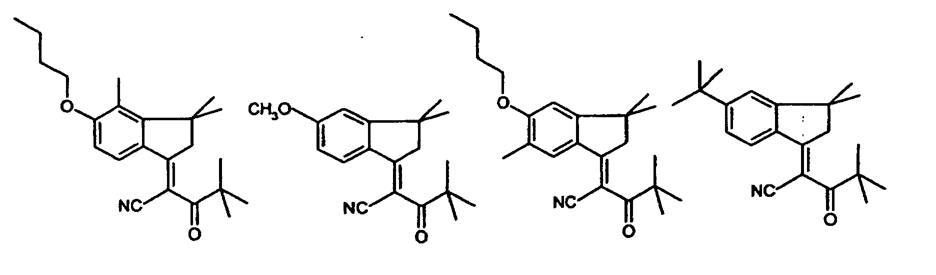

5. Kosmetische Mittel zum Schutz von Haut und Haaren nach Anspruch 3, enthaltend 2-(5,6-Dimethoxy-3,3-dimethyl-1-indanyliden)-4,4-dimethyl-3-oxo-pentansäurenitril, 2-(5-Methoxy-3,3,4,6-tetramethyl-1-indanyliden)-4,4-dimethyl-3-oxo-pentansäurenitril, 2-(3,3,5,6-tetramethyl-1-indanyliden)-4,4-dimethyl-3-oxo-pentansäurenitril, 2-(3,3,6-trimethyl-1-indanyliden)-4,4-dimethyl-3-oxo-pentansäurenitril, 2-(5,6-Ethylendioxo-3,3-dimethyl-1-indanyliden)-4,4-dimethyl-3-oxo-pentansäurenitril, 2-(5-Methoxy-3,3,6-trimethyl-1-indanyliden)-4,4-dimethyl-3-oxo-pentansäurenitril, 2-[(5-Methoxy-3,3-dimethyl-(2-methyl-3-(1,3,3,3-tetramethyl-1-(trimethyl-silyloxy)-disiloxanyl)-propy1)-indanyliden)]-4,4-dimethyl-3-oxo-pentan-säurenitril oder 2-(6-Acetoxy-3,3-dimethyl-5-methoxy-1-indanyliden)-4,4-dimethyl-3-oxo-pentansäurenitril.

6. Kosmetische Mittel nach Anspruch 3, **dadurch gekennzeichnet, dass** sie außerdem mindestens ein Bräunungs- und/oder künstliches Selbstbräunungsmittel für die Haut enthalten.

7. Kosmetische Mittel nach Anspruch 3, **dadurch gekennzeichnet, dass** sie neben den erfindungsgemäßen Verbindungen der Formel I einen oder mehrere weitere UV-Absorber enthalten.

8. Kosmetische Mittel nach Anspruch 3, **dadurch gekennzeichnet, dass** sie neben den erfindungsgemäßen Verbindungen der Formel I einen oder mehrere weitere UV-Absorber aus der Reihe Ethylhexylmethoxycinnamat, 2-Ethylhexyl-2-cyano-3,3-diphenylacrylat, 2-Phenylbenzimidazolsulfonsäure, Methylbenzylidencampher, 4-t-Butyl-4'-methoxy-dibenzoylmethan, Phenylen-bis-benzimidazyl-tetrasulfonsäure-dinatriumsalz, Terephthalyliden-dibomansulfonsäure und Salze, Phenol, 2-(2H-benzotriazol-2-yl)-4-methyl-6-(2-methyl-3(1,3,3,3-tetramethyl-1-(trimethylsilyl)-oxy)-disiloxyanyl)-propyl), Benzylidenmalonat-Polysiloxan, 2,2'-Methylen-bis-(6-(2H-benztriazol-2-yl)-4-1,1,3,3-tetramethylbutyl)-phenol), 2,4-Bis-[{(4-(2-Ethyl-hexyloxy-2-hydroxy}-phenyl]-6-(4-methoxyphenyl)-1,3,5-triazin, 4,4',4-(1,3,5-Triazin-2,4,6-triyltriimino)-tris-benzoesäure-tris(2-ethylhexylester), 4,4'-[(6-[4-(1,1-Dimethyl)-aminocarbonyl)-phenylamino]-1,3,5-triazin-2,4-diyl)diimino]-bis-(benzoesäure-2-ethylhexylester), Zinkoxid und Titandioxid enthalten.

9. Kosmetische Mittel nach Anspruch 3, **dadurch gekennzeichnet, dass** sie weitere UV-Absorber und zusätzlich umhüllte oder nicht umhüllte Pigmente oder Nanopigmente von Metalloxiden enthalten.

10. Kosmetische Mittel nach Anspruch 3, **dadurch gekennzeichnet, dass** sie weitere UV-Absorber und zusätzlich feinteilige Pigmente enthalten, so dass daraus eine UV-Breitbandschutzleistung mit einer kritischen Wellenlänge λcrit.> 380 nm resultiert.

11. Verwendung von Indanylidenverbindungen der Formel (I) aus Anspruch 1 als UV-Absorber in kosmetischen Mitteln zum Schutz für Haut und/oder der Haare gegen UV-Strahlung.

12. Verwendung von Indanylidenverbindungen der Formel (I) aus Anspruch 1 als UV-Absorber in Kombination mit UV-Absorbern des Typs Methoxycinnamat-Derivate oder des Typs Dibenzoylmethan-Derivate.

13. Verwendung von Indanylidenverbindungen der Formel (I) aus Anspruch 1 zur Photostabilisierung von UV-Absorbern des Typs Dibenzoylmethan-Derivate und Methoxycinnamate.

14. Verwendung von Indanylidenverbindungen der Formel (I) aus Anspruch 1 als UV-Absorber in Kombination mit einen oder mehreren UV-A-, UV-Bund/oder Breitbandfiltern als Einzelsubstanzen oder in Gemischen untereinander, in der Lipidphase und/oder in der wässrigen Phase.

15. Verwendung von Indanylidenverbindungen der Formel (I) aus Anspruch 1 zur Photostabilisierung von Kombinationen von UV-Absorbern des Typs der Methoxycinnamat-Derivate mit Dibenzoylmethan-Derivaten.

## Claims

1. Method for the preparation of indanylidene compounds of the formula 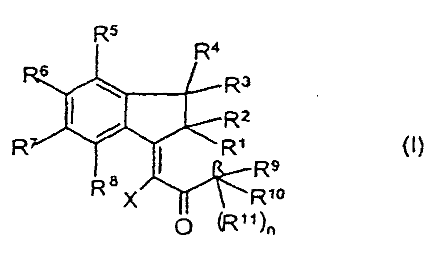 where
R¹ to R⁴ independently of one another denote hydrogen, C₁-C₂₀-alkyl or C₅-C₁₀-cycloalkyl, with the proviso that two substituents on adjacent C atoms together can also denote an optionally substituted C₁-C₄-alkylene group;
and furthermore independently of one another can denote C₂-C₂₀-alkyl, in which at least one methylene group can be replaced by oxygen, C₃-C₂₀-alkenyl, C3-C₂₀-alkynyl or a group S,
where S can be a silane, an oligosiloxane or a polysiloxane group;
R⁵ to R⁸ independently of one another denote hydrogen, C₁-C₂₀-alkyl or C₅-C₁₀-cylcloalkyl or C₁-C₂₀-alkoxy, C₅-C₁₀-cycloalkoxy, hydroxyl, acetoxy, acetamino, carboxyl, carbalkoxy or carbamoyl,
additionally two substituents of R⁵ to R⁸ on adjacent C atoms together can form a 5-7-membered ring, which contains up to three hetero-atoms, in particular oxygen or nitrogen, where the ring atoms can be substituted by exocyclically double-bonded oxygen (keto group).
and, furthermore, in the case of alkoxy, independently of one another can denote C₂-C₂₀-alkyl, in which at least one methylene group can be replaced by oxygen, C₃-C₂₀-alkenyl, C₃-C₂₀-alkynyl or a group S,
where S can be a silane, an oligosiloxane or a polysiloxane group;
X is cyano, CON(R)₂ or CO₂R, where R is hydrogen or C₁-C₈-alkyl;
n is 1 or 0;
if n = 1, R⁹ to R¹¹ can denote hydrogen, C₁-C₂₀-alkyl or C₅-C₁₀-cylcloalkyl, aryl or heteroaryl,
additionally two substituents of R⁹ to R¹¹ together with the β-atom can form a 3-7-membered ring, which can contain up to three hetero-atoms, in particular oxygen or nitrogen,
and, furthermore, if n = 0, R⁹ and R¹⁰ together with the β-atom denote an aryl or heteroaryl radical
and in which indanlylidene compounds of the formula (1) are obtained by (Knoevenagel) condensation of compounds of the formula 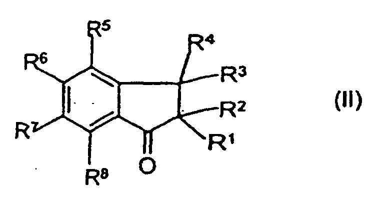 where R¹ to R⁸ have the abovementioned meaning,
with compounds of the formula 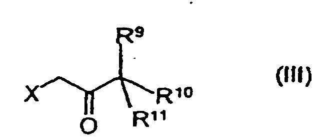 where R⁹ to R¹¹ and X have the abovementioned meaning.

2. Indanylidene compounds of the formula

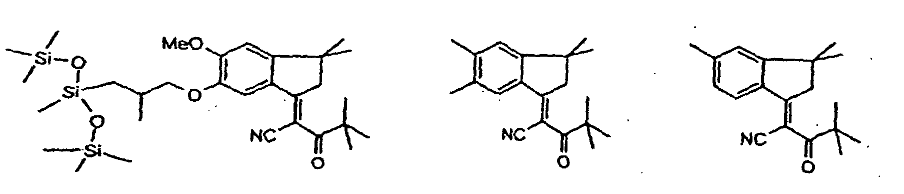

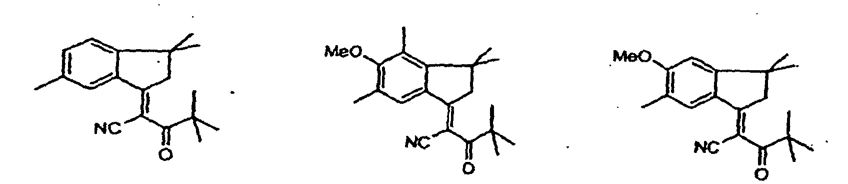

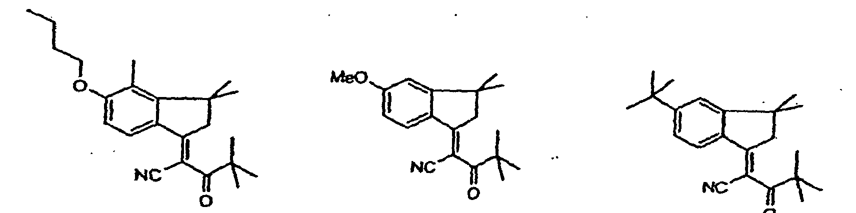

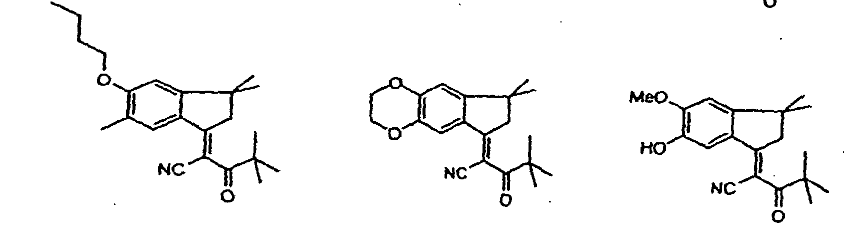

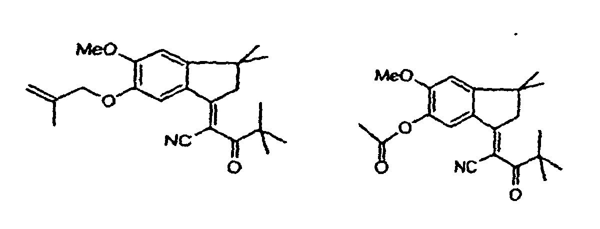

3. Cosmetic agents for the protection of skin and hair, containing indanylidene compounds of the formula 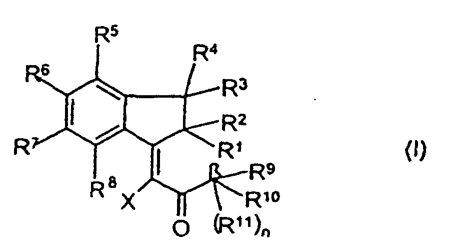 where
R¹ to R⁴ independently of one another denote hydrogen, C₁-C₂₀-alkyl or C₅-C₁₀-cycloalkyl, with the proviso that two substituents on adjacent C atoms together can also denote an optionally substituted C₁-C₄-alkylene group;
and furthermore independently of one another can denote C₂-C₂₀-alkyl, in which at least one methylene group can be replaced by oxygen, C₃-C₂₀-alkenyl, C₃-C₂₀-alkynyl or a group S,
where S can be a silane, an oligosiloxane or a polysiloxane group;
R⁵ to R⁸ independently of one another denote hydrogen, C₁-C₂₀-alkyl or C₅-C₁₀-cylcloalkyl or C₁-C₂₀-alkoxy, C₅-C₁₀-cycloalkoxy, hydroxyl, acetoxy, acetamino, carboxyl, carbalkoxy or carbamoyl,
additionally two substituents of R⁵ to R⁸ on adjacent C atoms together can form a 5-7-membered ring, which contains up to three hetero-atoms, in particular oxygen or nitrogen, where the ring atoms can be substituted by exocyclically double-bonded oxygen (keto group).
and, furthermore, in the case of alkoxy, independently of one another can denote C₂-C₂₀-alkyl, in which at least one methylene group can be replaced by oxygen, C₃-C₂₀-alkenyl, C₃-C₂₀-alkynyl or a group S,
where S can be a silane, an oligosiloxane or a polysiloxane group;
X is cyano, CON(R)₂ or CO₂R, where R is hydrogen or C₁-C₈-alkyl;
n is 1 or 0;
if n = 1, R⁹ to R¹¹ can denote hydrogen, C₁-C₂₀-alkyl or C₅-C₁₀-cylcloalkyl, aryl or heteroaryl,
additionally two substituents of R⁹ to R¹¹ together with the β-atom can form a 3-7-membered ring, which can contain up to three hetero-atoms, in particular oxygen or nitrogen,
and, furthermore, if n = 0, R⁹ and R¹⁰ together with the β-atom denote an aryl or heteroaryl radical

4. Cosmetic agents for the protection of skin and hair according to Claim 3, containing

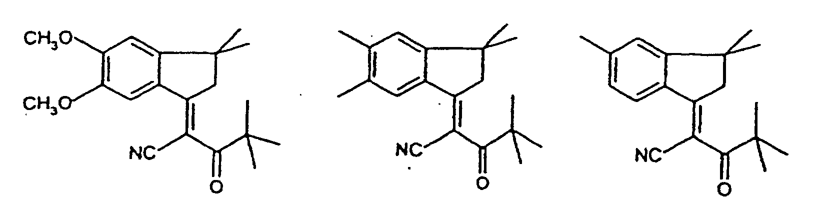

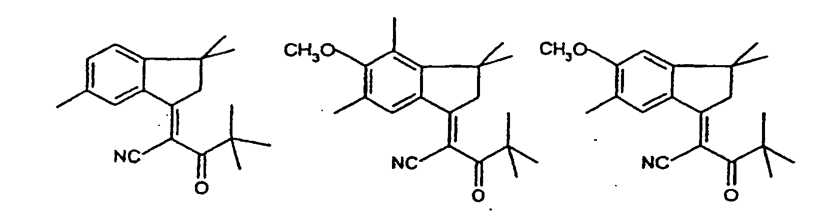

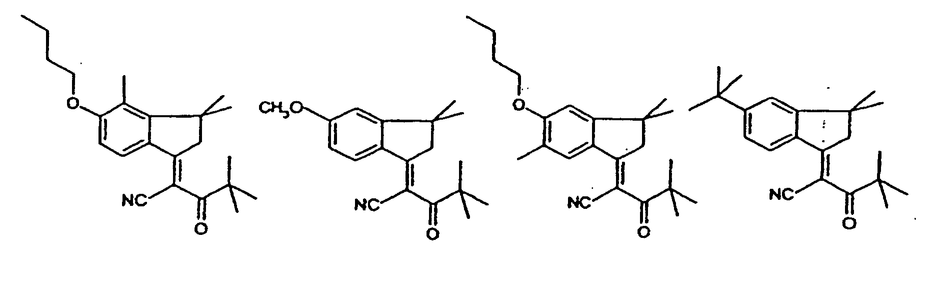

5. Cosmetic agents for the protection of skin and hair according to Claim 3, containing 2-(5,6-dimethoxy-3,3-dimethyl-1-indanylidene)-4,4-dimethyl-3-oxo-pentanoic acid nitrile, 2-(5-methoxy-3,3,4,6-tetramethyl-1-indanylidene)-4,4-dimethyl-3-oxo-pentanoic acid nitrile, 2-(3,3,5,6-tetramethyl-1-indanylidene)-4,4-dimethyl-3-oxo-pentanoic acid nitrile, 2-(3,3,6-trimethyl-1-indanylidene)-4,4-dimethyl-3-oxo-pentanoic acid nitrile, 2-(5,6-ethylenedioxo-3,3-dimethyl-1-indanylidene)-4,4-dimethyl-3-oxo-pentanoic acid nitrile, 2-(5-methoxy-3,3,6-trimethyl-1-indanylidene)-4,4-dimethyl-3-oxo-pentanoic acid nitrile, 2-[(5-methoxy-3,3-dimethyl-(2-methyl-3-(1,3,3,3-tetramethyl-1-(trimethyl-silyl-oxy)-disiloxanyl)-propyl (sic))-indanylidene)]-4,4-dimethyl-3-oxo-pentanoic acid nitrile or 2-(6-actoxy-3,3-dimethyl-5-methoxy-1-indanylidene)-4,4-dimethyl-3-oxo-pentanoic acid nitrile.

6. Cosmetic agents according to Claim 3, **characterised in that** they also contain at least one tanning and/or synthetic self-tanning agent for the skin.

7. Cosmetic agents according to Claim 3, **characterised in that**, in addition to the compounds of the Formula I according to the invention, they contain one or more further UV absorbers.

8. Cosmetic agents according to Claim 3, **characterised in that**, in addition to the compounds of the Formula I according to the invention, they contain one or more further UV absorbers from the series ethylhexyl methoxycinnamate, 2-ethylhexyl 2-cyano-3,3-diphenylacrylate, 2-phenylbenzimidazolesulphonic acid, methylbenzylidenecamphor, 4-t-butyl-4-methoxy-dibenzoylmethane, disodium phenylene-bis-benzimidazyl-tetrasulphonate, terephthalylidene-dibomanesulphonic acid and salts, phenol, 2-(2H-benzotriazol-2-yl)-4-methyl-6-(2-methyl-3(1,3,3,3-tetramethyl-1-(trimethylsilyl)-oxy)-disiloxyanyl)-propyl), benzylidene malonate polysiloxane, 2,2'-methylene-bis-(6-(2H-benztraizol-2-yl)-4-[lacuna] 1,1,3,3-tetramethylbutyl)-phenol), 2,4-bis[ {(4-(2-ethyl-hexyloxy-2-hydroxy}-phenyl]-6-(4-methoxyphenyl)-1,3,5-triazine (sic), tris(2-ethylhexyl) 4,4',4-(1,3,5-triazine-2,4,6-triyltriimino)-tris-benzoate, 4,4'-[(6-[4-(1,1-dimethyl)-aminocarbonyl)-phenylamino]-1,3,5-triazine-2,4-diyl)diimino]-bis-(2-ethylhexyl benzoate), zinc oxide and titanium dioxide.

9. Cosmetic agents according to Claim 3, **characterised in that** they contain further UV absorbers and additionally contain coated or non-coated pigments or nanopigments of metal oxides.

10. Cosmetic agents according to Claim 3, **characterised in that** they contain further UV absorbers and additionally contain finely divided pigments, so that a UV broadband protection with a critical wavelength λcrit > 380 nm results therefrom.

11. Use of indanylidene compounds of the Formula (I) from Claim I as UV absorbers in cosmetic agents for the protection for skin and/or hair from UV radiation.

12. Use of indanylidene compounds of the Formula (1) from Claim 1 as UV absorbers in combination with UV absorbers of the methoxycinnamate derivatives type or of the dibenzoylmethane derivatives type.

13. Use of indanylidene compounds of the Formula (I) from Claim 1 for the photostabilisation of UV absorbers of the dibenzoylmethane derivatives and methoxycinnamates type

14. Use of indanylidene compounds of the Formula (I) from Claim 1 as UV absorbers in combination with one or more UV-A, UV-B and/or broadband filters as individual substances or in mixtures with one another, in the lipid phase and/or in the aqueous phase.

15. Use of indanylidene compounds of the Formula (I) from Claim 1 for the photostabilisation of combinations of UV absorbers of the methoxycinnamate derivatives with dibenzoylmethane derivatives type.

## Revendications

1. Procédé de préparation de composés indanylidène de la formule : 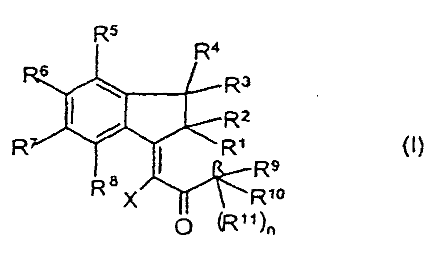 où
R¹ à R⁴ représentent indépendamment l'un de l'autre, hydrogène, alkyle en C₁-C₂₀ ou cycloalkyle en C₅-C₁₀, avec la condition que également, deux substituants sur des atomes C voisins peuvent représenter ensemble, un radical alkylène en C₁-C₄ le cas échéant substitué ;
peuvent représenter d'autre part, indépendamment l'un de l'autre, alkyle en C₂-C₂₀, dans lequel au moins un radical méthylène peut être remplacé par oxygène, alcényle en C₃-C₂₀, alcynyle en C₃-C₂₀ ou un radical S,
où S peut être un radical silane, oligosiloxane ou polysiloxane ;
R⁵ à R⁸ représentent indépendamment l'un de l'autre, hydrogène, alkyle en C₁-C₂₀ ou cycloalkyle en C₅-C₁₀ ou alcoxy en C₁-C₂₀, cycloalcoxy en C₅-C₁₀, hydroxy, acétoxy, acétamino, carboxy, carbalcoxy ou carbamoyle,
en outre deux substituants de R⁵ à R⁸ sur des atomes C voisins peuvent former ensemble, un cycle de 5 à 7 membres, qui contient jusqu'à 3 hétéroatomes, en particulier l'oxygène ou l'azote, où les atomes cycliques peuvent être substitués par de l'oxygène lié doublement de manière exocyclique (radical céto),
d'autre part, dans le cas de alcoxy, ils peuvent représenter indépendamment l'un de l'autre, un alkyle en C₂-C₂₀ dans lequel au moins un radical méthylène peut être remplacé par oxygène, alcényle en C₃-C₂₀, alcynyle en C₃-C₂₀ ou un radical S,
où S peut être un radical silane, oligosiloxane ou polysiloxane ;
X représente cyano, CON(R)₂ ou CO₂R, où R représente hydrogène ou alkyle en C₁-C₈,
n représente 1 ou 0 ;
R⁹ à R¹¹ peuvent représenter dans le cas de n = 1, hydrogène, alkyle en C₁-C₂₀ ou cycloalkyle en C₅-C₁₀, aryle ou hétéroaryle,
en outre deux substituants de R⁹ à R¹¹ peuvent former avec l'atome β, un cycle de 3 à 7 membres, qui peut contenir jusqu'à 3 hétéroatomes, en particulier l'oxygène ou l'azote,
d'autre part, dans le cas de n = 0, R⁹ à R¹¹ représentent avec l'atome β, un reste aryle ou hétéroaryle,
et dans lequel les composés indanylidène de la formule (I) sont obtenus par condensation (de Knoevenagel) de composés de la formule : 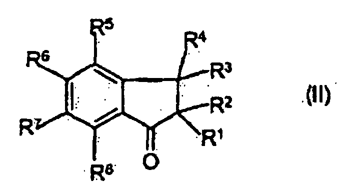 où R¹ à R⁸ ont la signification donnée ci-dessus,
avec des composés de la formule : 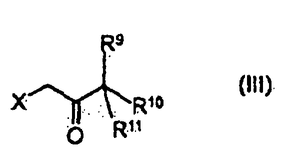 où R⁹ à R¹¹ ont la signification donnée ci-dessus.

2. Composés indanylidène de la formule :

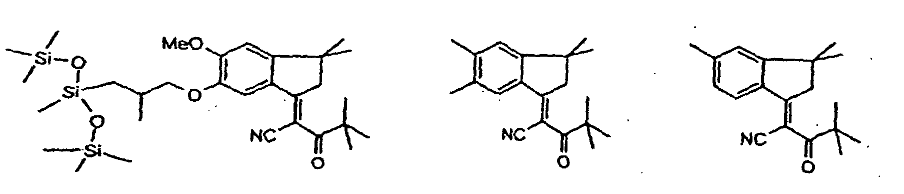

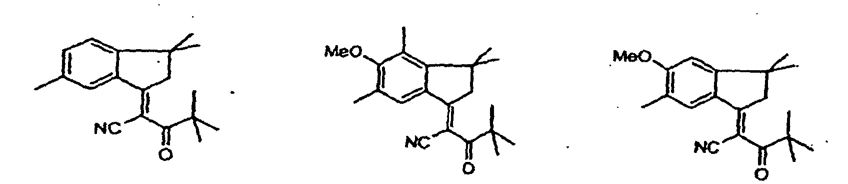

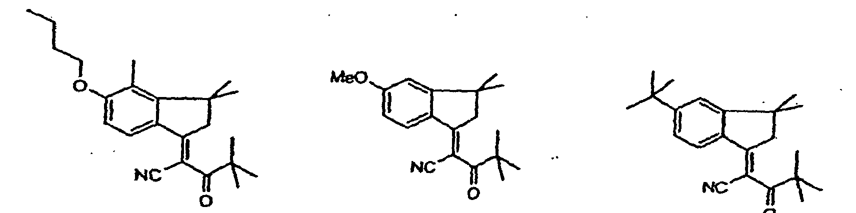

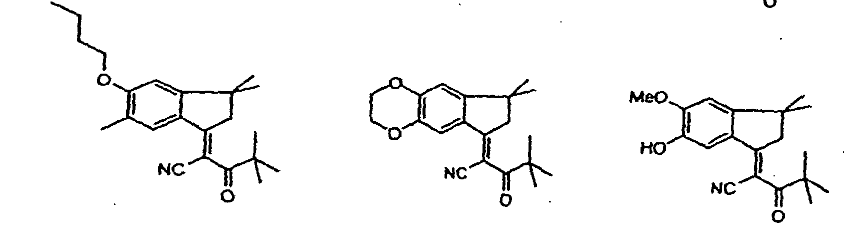

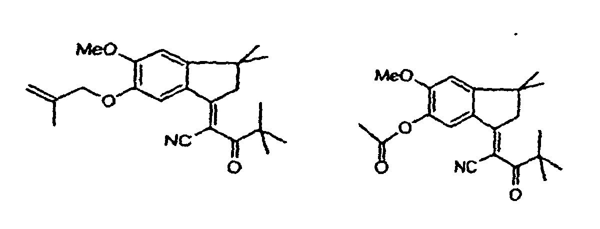

3. Agent cosmétique pour la protection de la peau et des cheveux, contenant des composés indanylidène de la formule : 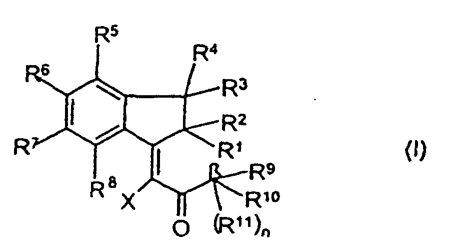 où
R¹ à R⁴ représentent indépendamment l'un de l'autre, hydrogène, alkyle en C₁-C₂₀ ou cycloalkyle en C₅-C₁₀, avec la condition que également, deux substituants sur des atomes C voisins peuvent représenter ensemble, un radical alkylène en C₁-C₄ le cas échéant substitué ;
peuvent représenter d'autre part, indépendamment l'un de l'autre, alkyle en C₂-C₂₀, dans lequel au moins un radical méthylène peut être remplacé par oxygène, alcényle en C₃-C₂₀, alcynyle en C₃-C₂₀ ou un radical S,
où S peut être un radical silane, oligosiloxane ou polysiloxane ;
R⁵ à R⁸ représentent indépendamment l'un de l'autre, hydrogène, alkyle en C₁-C₂₀ ou cycloalkyle en C₅-C₁₀ ou alcoxy en C₁-C₂₀, cycloalcoxy en C₅-C₁₀, hydroxy, acétoxy, acétamino, carboxy, carbalcoxy ou carbamoyle,
en outre deux substituants de R⁵ à R⁸ sur des atomes C voisins peuvent former ensemble, un cycle de 5 à 7 membres, qui contient jusqu'à 3 hétéroatomes, en particulier l'oxygène ou l'azote, où les atomes cycliques peuvent être substitués par de l'oxygène lié doublement de manière exocyclique (radical céto),
d'autre part, dans le cas de alcoxy, ils peuvent représenter indépendamment l'un de l'autre, un alkyle en C₂-C₂₀ dans lequel au moins un radical méthylène peut être remplacé par oxygène, alcényle en C₃-C₂₀, alcynyle en C₃-C₂₀ ou un radical S,
où S peut être un radical silane, oligosiloxane ou polysiloxane ;
X représente cyano, CON(R)₂ ou CO₂R, où R représente hydrogène ou alkyle en C₁-C₈,
n représente 1 ou 0 ;
R⁹ à R¹¹ peuvent représenter dans le cas de n = 1, hydrogène, alkyle en C₁-C₂₀ ou cycloalkyle en C₅-C₁₀, aryle ou hétéroaryle,
en outre deux substituants de R⁹ à R¹¹ peuvent former avec l'atome β, un cycle de 3 à 7 membres, qui peut contenir jusqu'à 3 hétéroatomes, en particulier l'oxygène ou l'azote,
d'autre part, dans le cas de n = 0, R⁹ à R¹¹ représentent avec l'atome β, un reste aryle ou hétéroaryle.

4. Agent cosmétique pour la protection de la peau et des cheveux selon la revendication 3, contenant :

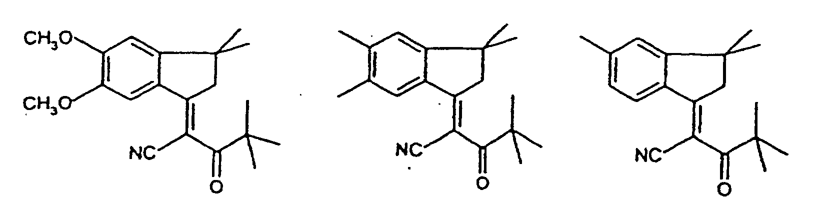

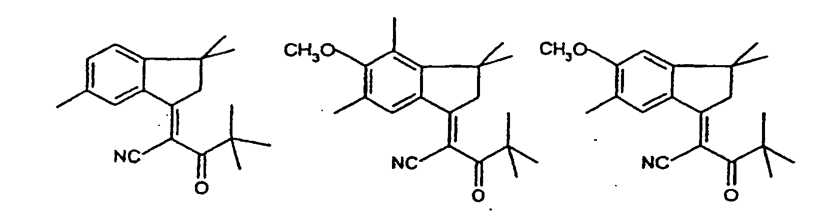

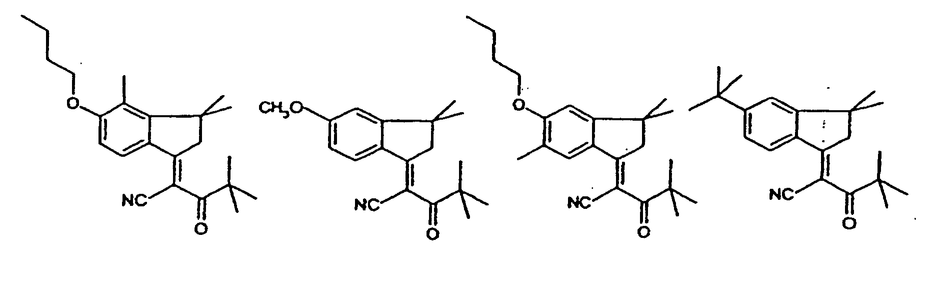

5. Agent cosmétique pour la protection de la peau et des cheveux selon la revendication 3, contenant le nitrile de l'acide 2- (5,6-diméthoxy-3,3-diméthyl-1-indanylidène)-4,4-diméthyl-3-oxopentanoïque, le nitrile de l'acide 2-(5-méthoxy-3, 3, 4 , 6-tétraméthyl-1-indanylidène)-4,4-diméthyl-3-oxopentanoique, le nitrile de l'acide 2-(3,3,5,6-tétraméthyl-1-indanylidène)-4,4-diméthyl-3-oxopentanoïque, le nitrile de l'acide 2-(3,3,6-triméthyl-1-indanylidène)-4,4-diméthyl-3-oxo-pentanoïque, le nitrile de l'acide 2-(5,6-éthylènedioxo-3,3,6-triméthyl-1-indanylidène) -4,4-diméthyl-3-oxopentanoique, le nitrile de l'acide 2-[(5-méthoxy-3,3-diméthyl-(2-méthyl-3-(1,3,3,3-tétraméthyl-1-(triméthylsilyloxy)disiloxanyl)propyl)-1-indanylidène)]-4,4-diméthyl-3-oxopentanoïque ou le nitrile de l'acide 2-(6-acétoxy-3,3-diméthyl-5-méthoxy-1-indanylidène)-4 , 4-ditnéthyl-3-oxopentanoïque.

6. Agent cosmétique selon la revendication 3, **caractérisé en ce qu'**ils contient en outre, au moins un agent de bronzage et/ou d'auto-bronzage artificiel, pour la peau.

7. Agent cosmétique selon la revendication 3, **caractérisé en ce qu'**il contient en plus des composés de la formule I suivant l'invention, un ou plusieurs autres agents absorbant les U.V.

8. Agent cosmétique selon la revendication 3, **caractérisé en ce qu'**il contient en plus des composés de la formule I suivant l'invention, un ou plusieurs autres agents absorbant les U.V. de la série du méthoxycinnamate d'éthylhexyle, du 2-cyano-3,3-diphénylacrylate de 2-éthylhexyle, de l'acide 2-phénylbenzimidazolesulfonique, du méthylbenzylidènecamphre, du 4-t-butyl-4'-méthoxydibenzoylméthane, du sel disodique de l'acide phénylène-bis-benzimidazyltétrasulfonique, de l'acide téréphtalylidènedibornanesulfonique et des sels, du phénol, du 2-(2H-benzotriazol-2-yl)-4-méthyl-6-(2-méthyl-3-(1,3,3,3-tétraméthyl-1-(triméthylsilyl)oxy)disiloxanyl)propyle), du malonate de benzylidène-polysiloxane, du 2,2'-méthylène-bis(6-(2H-benztriazol-2-yl)-4-(1,1,3,3-tétraméthyl)butyl)phénol), de la 2,4-bis[{(4- (2-éthyl)hexyloxy-2-hydroxy}phényl]-6-(4-méthoxyphényl)-1,3,5-triazine, de l'ester tris(2-éthylhexylique) de l'acide 4,4',4-(1,3,5-triazine-2,4,6-triyltriimino)trisbenzoique, de l'ester bis (2-éthylhexylique) de l'acide 4,4'-[(6-[4-(1,1-diméthyl)aminocarbonyl)phénylamino]-(1,3,5-triazine-2,4-diyl)diimino]bisbenzoïque, de l'oxyde de zinc et du dioxyde de titane.

9. Agent cosmétique selon la revendication 3, **caractérisé en ce qu'**il contient d'autres agents absorbant les U.V. et en outre, des pigments enrobés ou non enrobés ou des nanopigments d'oxydes métalliques.

10. Agent cosmétique selon la revendication 3, **caractérisé en ce qu'**il contient d'autres agents absorbant les U.V. et en outre, des pigments fins, de sorte qu'il en résulte une capacité de protection à large bande contre les U.V. avec une longueur d'onde critique λ_{crit} > 380 nm.

11. Utilisation des composés indanylidène de la formule (I) selon la revendication 1, comme agent absorbant les U.V. dans des agents cosmétiques pour protéger la peau et/ou les cheveux contre le rayonnement U.V.

12. Utilisation des composés indanylidène de la formule (I) selon la revendication 1, comme agent absorbant les U.V., en combinaison avec des agents absorbant les U.V. de type dérivés de méthoxycinnamate ou de type dérivés de dibenzoylméthane.

13. Utilisation des composés indanylidène de la formule (I) selon la revendication 1, pour la photostabilisation des agents absorbant les U.V. de type dibenzoylméthane et méthoxycinnamate.

14. Utilisation des composés indanylidène de la formule (I) selon la revendication 1, comme agent absorbant les U.V., en combinaison avec un ou plusieurs filtres U.V.-A, U.V.-B et/ou à large bande, comme substance unique ou en mélange, en phase lipidique et/ou en phase aqueuse.

15. Utilisation des composés indanylidène de la formule (I) selon la revendication 1, pour la photostabilisation de combinaisons d'agents absorbant les U.V. de type dérivés méthoxycinnamate avec des dérivés dibenzoylméthane.
